(19) **Europäisches Patentamt / European Patent Office / Office européen des brevets**

(11) **EP 4 678 093 A2**

(12) # EUROPEAN PATENT APPLICATION

(43) Date of publication:
**14.01.2026 Bulletin 2026/03**

(21) Application number: **25219710.8**

(22) Date of filing: **02.05.2022**

(51) International Patent Classification (IPC):
**A61B 5/00** *(2006.01)*

(52) Cooperative Patent Classification (CPC):
**A61B 5/02028; A61B 5/0205; A61B 5/021;
A61B 5/14551; A61B 5/7203; A61B 5/7275**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **18.05.2021 US 202163189813 P
10.08.2021 US 202163231463 P**

(62) Document number(s) of the earlier application(s) in
accordance with Art. 76 EPC:
**22728997.2 / 4 312 738**

(71) Applicant: **Becton, Dickinson and Company
Franklin Lakes, NJ 07417-1880 (US)**

(72) Inventors:
  • **ALATHUR RANGARAJAN, Anusha
    Irvine, CA, 92614 (US)**
  • **BENNI, Paul, B.
    Irvine, CA, 92614 (US)**

  • **JIAN, Zhongping
    Irvine, CA, 92614 (US)**
  • **ALBANESE, Antonio
    Irvine, CA, 92614 (US)**
  • **AGUIRRE, Andres, S.
    Irvine, CA, 92614 (US)**
  • **SCHNEIDER, Brennan, Michael
    Irvine, CA, 92614 (US)**

(74) Representative: **Venner, Julia Ann et al
Kilburn & Strode LLP
Lacon London
84 Theobalds Road
London WC1X 8NL (GB)**

Remarks:
This application was filed on 01.12.2025 as a
divisional application to the application mentioned
under INID code 62.

(54) **AUTOREGULATION SYSTEM AND METHOD USING TISSUE OXIMETRY AND BLOOD PRESSURE**

(57)      A method and apparatus for determining a subject's autoregulation function state is provided. The method includes: continuously sensing a tissue region of a subject with a tissue oximeter to produce first signals representative of at least one tissue oxygenation parameter during a period of time; continuously measuring a blood pressure level of the subject during the period of time to produce second signals representative of the subject's blood pressure during the period of time; determining a presence of a confounding factor that affects the sensed tissue oxygenation parameter in a manner independent of an autoregulation function of the subject, the determination using the first signals; using the first and second signals to determine an autoregulation function state of the subject when the absence of the confounding factor is determined. The method may include determining an at least one of an LLA or a ULA of a subject's autoregulation function state.

*FIG. 4A*

EP 4 678 093 A2

**Description**

This application claims priority based on United States Provisional Patent Application Serial No. 63/189,813, filed May 18, 2021 and entitled AUTOREGULATION SYSTEM AND METHOD USING TISSUE OXIMETRY AND BLOOD PRESSURE, and on United States Provisional Patent Application Serial No. 63/231,463, filed August 10, 2021 and entitled AUTOREGULATION SYSTEM AND METHOD USING TISSUE OXIMETRY AND BLOOD PRESSURE, the complete disclosures of both of which are hereby incorporated by reference herein in their entireties.

BACKGROUND OF THE INVENTION

1. Technical Field

The present disclosure relates to medical apparatus and methods in general, and to medical apparatus and methods for measuring and/or monitoring autoregulation in particular.

2. Background Information

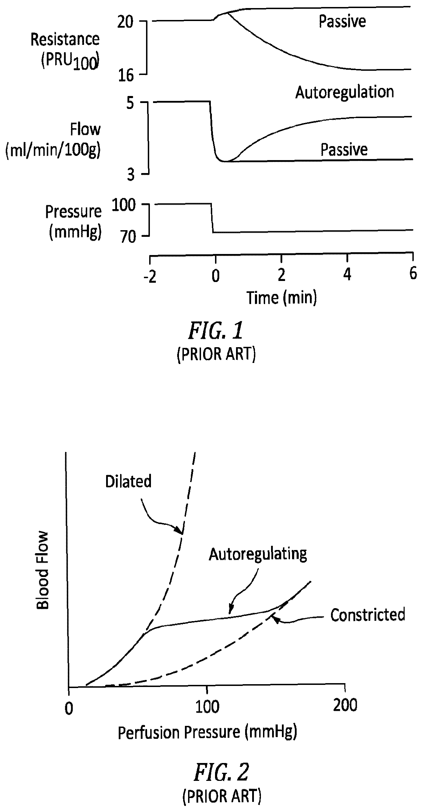

Autoregulation is a process in mammals that aims to maintain adequate and stable (e.g., "constant") blood flow to organs (e.g., brain, heart, kidneys, etc.) for a range of perfusion pressures. While most systems of the body show some degree of autoregulation, the brain is very sensitive to overperfusion as well as underperfusion. FIG. 1 shows the effects of suddenly reducing perfusion pressure from 100 to 70 mmHg. In a passive vascular bed (i.e., poor autoregulation), this sudden drop in pressure will result in a rapid and sustained fall in blood flow. With autoregulation, vascular resistance is increased, in an effort to return to nominal flow. The range that vascular resistance can vary has limitations, however. Arterial blood vessels can reach a point of maximum dilation due to a vasodilator drug or other cause, in which vascular reactivity (i.e., the ability to change vascular resistance) becomes passive. In a passive state, a change in blood pressure may result in a change in blood flow. If the blood flow decreases sufficiently, inadequate perfusion and resultant ischemia within the organ may occur. Conversely, arterial blood vessels can reach a state of maximum constriction, in which vascular reactivity also becomes passive. Increased blood pressure can result in excessive flow to the organ; e.g., see FIG. 2.

Different organs display varying degrees of autoregulatory behavior. The renal, cerebral, and coronary circulations typically show excellent autoregulation, whereas skeletal muscle and splanchnic circulations show moderate autoregulation. The cutaneous circulation shows little or no autoregulatory capacity.

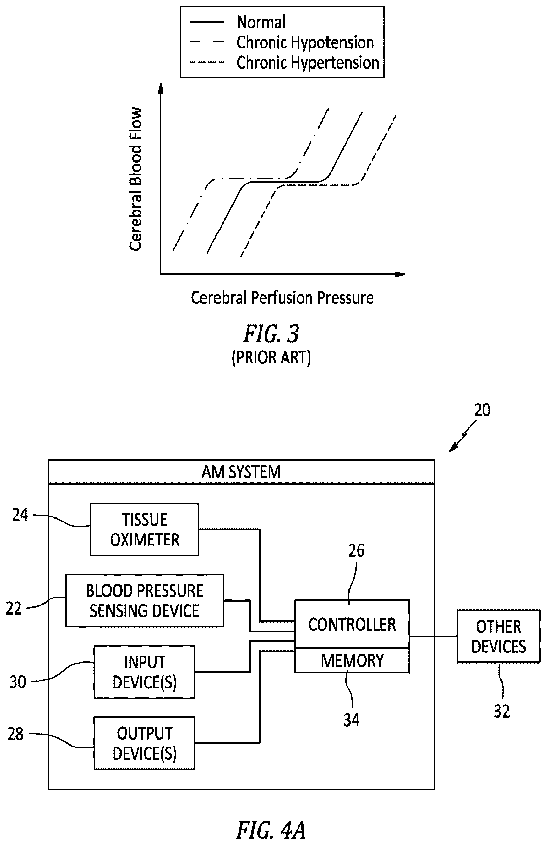

A plurality of factors (e.g., a hardening of the arteries that occurs with advancing age) can change the characteristics of a vascular reactivity response, and these factors can in turn change relevant autoregulation characteristics. Hence, the autoregulation range of blood flow due to changing blood pressure can vary between subjects and cannot assumed to be a constant. FIG. 3 illustrates how a cerebral autoregulation curve can shift due to chronic hypertension and hypotension. Methods and apparatus for determining whether a particular subject's autoregulation is functioning, and the potential range to manage blood pressure variability, would be a great help to a clinician.

Furthermore, in some instances the physiological data that is used to determine or measure a subject's autoregulation state may be influenced by factors independent of the subject's autoregulation system. For example, one or more NIRS indices (e.g., tissue oxygen saturation ($StO_2$), relative total hemoglobin concentration per volume of tissue (rTHb), differential changes in oxyhemoglobin (O2Hb) and deoxyhemoglobin (HHb), HbD (i.e., O2Hb - HHb), etc.) may be at a level that is not attributable to autoregulation. In these instances, an autoregulation determination or measurement made using these values may negatively affect the accuracy of the determination or measurement. As another example, if a subject's blood carbon dioxide level is outside of a normal range (normocapnia), the accuracy of an autoregulation determination or measurement may be negatively affected.

What is needed is an apparatus and method for monitoring autoregulation that is an improvement over those known in the prior art, including one that identifies and accounts for factors that may confound an autoregulation determination or measurement.

SUMMARY

According to an aspect of the present disclosure, a method for determining a subject's autoregulation function state is provided. The method includes: continuously sensing a tissue region of a subject with a tissue oximeter, the sensing producing first signals representative of at least one tissue oxygenation parameter during a period of time; continuously measuring a blood pressure level of the subject using a blood pressure sensing device during the period of time, the measuring producing second signals representative of the blood pressure of the subject during the period of time; determining a presence or an absence of a confounding factor that affects the sensed at least one tissue oxygenation parameter in a manner independent of an autoregulation function of the subject, the determination using the first signals; and using the first signals and the second signals to determine an autoregulation function state of the subject when the absence of the confounding factor is determined.

In any of the aspects or embodiments described above and herein, the step of determining the presence or absence of the confounding factor may further include determining whether the confounding factor has affected the measured blood pressure level of the subject in a manner independent of the autoregulation function of the subject using the second signals.

In any of the aspects or embodiments described above and herein, the step of determining the presence or absence of the confounding factor may utilize a tissue oxygenation parameter trend data based on the first signals, and a blood pressure level trend data based on the second signals.

In any of the aspects or embodiments described above and herein, the method may further include determining a heart rate of the subject during the period of time, and producing third signals representative of the subject's heart rate during the period of time, and the step of determining the presence or absence of the confounding factor further may include determining whether the confounding factor has affected the heart rate of the subject in a manner independent of the autoregulation function of the subject using the third signals.

In any of the aspects or embodiments described above and herein, the step of determining the presence or absence of the confounding factor may utilize a tissue oxygenation parameter trend data based on the first signals, and a heart rate trend data based on the third signals.

In any of the aspects or embodiments described above and herein, the step of determining the presence or absence of the confounding factor further may include evaluating the first signals using a magnitude of change filter.

In any of the aspects or embodiments described above and herein, the at least one tissue oxygenation parameter may include one or more of tissue oxygen saturation ($StO_2$), total hemoglobin concentration per volume of tissue (THb), relative total hemoglobin concentration per volume of tissue (rTHb), differential changes in oxyhemoglobin (O2Hb) and deoxyhemoglobin (HHb), or O2Hb - HHb (HbD).

In any of the aspects or embodiments described above and herein, the step of determining the presence or absence of the confounding factor may further include determining a blood carbon dioxide ($CO_2$) level of the subject.

In any of the aspects or embodiments described above and herein, the at least one tissue oxygenation parameter may include a first tissue oxygenation parameter and a second oxygenation parameter, and the first signals produced from the sensing may include a first subset of first signals representative of the first tissue oxygenation parameter, and a second subset of first signals representative of the second tissue oxygenation parameter, and the step of determining the presence or absence of the confounding factor may utilize the first subset of first signals and the second subset of first signals.

In any of the aspects or embodiments described above and herein, the first tissue oxygenation parameter may be one of $StO_2$, THb, rTHb, differential changes in O2Hb and HHb, or HbD, and the second tissue oxygenation parameter may be another of $StO_2$. THb, rTHb, differential changes in O2Hb and HHb, or HbD.

In any of the aspects or embodiments described above and herein, the step of determining the presence or absence of the confounding factor may utilize a first tissue oxygenation parameter trend data based on the first subset of first signals, and a second tissue oxygenation parameter trend data based on the second subset of first signals.

In any of the aspects or embodiments described above and herein, the tissue oximeter may be a near-infrared spectroscopy (NIRS) type tissue oximeter.

In any of the aspects or embodiments described above and herein, the tissue being continuously sensed may be brain tissue, and the autoregulation function state determined in the absence of the confounding factor may be a brain autoregulation function state of the subject.

In any of the aspects or embodiments described above and herein, the step of determining the presence or absence of the confounding factor further may include evaluating the first signals to determine extracerebral blood flow as the confounding factor.

In any of the aspects or embodiments described above and herein, the step of continuously sensing the tissue region of the subject with the tissue oximeter may include using one or more sensors in communication with the tissue oximeter, the one or more sensors each having at least one light source, at least one near detector located a first distance from the at least one light source, and at least one far detector located a second distance from the at least one light source, where the second distance is greater than the first distance.

According to another aspect of the present disclosure, an apparatus for determining a subject's autoregulation function state is provided. The apparatus includes a near infra-red spectroscopy (NIRS) tissue oximeter, a blood pressure sensing device, and a controller. The NIRS tissue oximeter is configured to continuously sense a tissue region of the subject during a period of time, and to produce first signals representative of at least one tissue oxygenation parameter during the period of time. The blood pressure sensing device is configured to continuously measure a blood pressure level of the subject during the period of time, and to produce second signals representative of the blood pressure of the subject during the period of time. The controller is in communication with the NIRS tissue oximeter and the blood pressure sensing device. The controller includes at least one processor and a memory device configured to store instructions. The stored instructions when executed cause the controller to: control the NIRS tissue oximeter to continuously sense a tissue region of the subject during a period of time, and to produce first signals representative of at least one tissue oxygenation parameter sensed within the tissue region during the period of time; control the blood pressure sensing device to continuously measure a blood pressure level of the subject during the period of time, and to produce second signals representative of the blood pressure of the subject during the period of time; determine a presence or an absence of a confounding factor using the first signals, the confounding factor operable to affect the sensed at least one tissue oxygenation parameter in a manner independent of an autoregulation function of the subject; and using the first signals and the second signals, determine an autoregulation function state of the subject when the absence of the confounding factor is determined.

In any of the aspects or embodiments described above and herein, the stored instructions when executed may cause the at least one processor to determine the autoregulation function state of the subject without using the first signals and the second signals when the presence of the confounding factor is determined.

In any of the aspects or embodiments described above and herein, the stored instructions when executed may cause the at least one processor to determine the autoregulation function state of the subject using the first signals and the second signals when the presence of the confounding factor is determined, and to flag the autoregulation function state.

In any of the aspects or embodiments described above and herein, the stored instructions when executed may cause the controller to determine whether the confounding factor has affected the measured blood pressure level of the subject in a manner independent of the autoregulation function of the subject using the second signals.

In any of the aspects or embodiments described above and herein, the stored instructions when executed may cause the controller to determine a tissue oxygenation parameter trend data based on the first signals and to determine a blood pressure level trend data based on the second signals, and the determination of said presence or said absence of the confounding factor utilizes the tissue oxygenation parameter trend data and the blood pressure level trend data.

In any of the aspects or embodiments described above and herein, the stored instructions when executed may cause the controller to determine a heart rate of the subject during the period of time, and to produce third signals representative of the subject's heart rate during the period of time, and the stored instructions when executed may cause the controller to determine whether the confounding factor has affected the heart rate of the subject in a manner independent of the autoregulation function of the subject using the third signals.

In any of the aspects or embodiments described above and herein, the stored instructions when executed may cause the controller to determine a tissue oxygenation parameter trend data based on the first signals, and a heart rate trend data based on the third signals, and the determination of said presence or said absence of the confounding factor utilizes the tissue oxygenation parameter trend data and the heart rate trend data.

In any of the aspects or embodiments described above and herein, the stored instructions when executed may cause the controller to evaluate the first signals using a magnitude of change filter.

In any of the aspects or embodiments described above and herein, the apparatus may further include a $CO_2$ sensor configured to sense a blood carbon dioxide ($CO_2$) level of the subject, and the instructions when executed may cause the controller to determine a blood carbon dioxide ($CO_2$) level of the subject using the $CO_2$ sensor.

In any of the aspects or embodiments described above and herein, the tissue oximeter may be configured to sense extracerebral tissue and cerebral tissue, and the stored instructions when executed may cause the tissue oximeter to continuous sense extracerebral tissue and brain tissue, and the autoregulation function state determined in the absence of the confounding factor may be a brain autoregulation function state of the subject.

In any of the aspects or embodiments described above and herein, the stored instructions when executed may cause the controller to determine extracerebral blood flow as a confounding factor.

In any of the aspects or embodiments described above and herein, the tissue oximeter may include one or more sensors each having at least one light source, at least one near detector located a first distance from the at least one light source, and at least one far detector located a second distance from the at least one light source, where the second distance is greater than the first distance.

According to an aspect of the present disclosure, a method for determining at least one of a lower limit of autoregulation (LLA) or an upper limit of autoregulation (ULA) of a subject's autoregulation function state. The method includes: a) continuously sensing a tissue region of a subject with a tissue oximeter, the sensing producing first signals representative of at least one tissue oxygenation parameter during a period of time; b) continuously measuring a blood pressure level of the subject using a blood pressure sensing device during the period of time, the measuring producing second signals representative of the blood pressure of the subject during the period of time; c) determining autoregulation data as a function of subject blood pressure using the first signals representative of at least one tissue oxygenation parameter and the second signals representative of the blood pressure of the subject during the period of time; and d) determining at least one of a lower limit of autoregulation or an upper limit of autoregulation of the subject's autoregulation function state.

In any of the aspects of embodiments described above or herein, the step of determining at least one of an LLA or a ULA of the subject's autoregulation function state may include fitting a curve to the autoregulation data as a function of subject blood pressure using an algorithm.

In any of the aspects or embodiments described above and herein, the step of fitting the curve to the autoregulation data may include determining an algorithmic model of the curve, and the step of determining at least one of the LLA or the ULA of the subject's autoregulation function state using the fitted curve may include determining an inflection point using the algorithmic model of the curve.

In any of the aspects or embodiments described above and herein, wherein the determination of the inflection point may include determining a first derivative of the curve.

In any of the aspects or embodiments described above and herein, wherein the determination of the inflection point may use at least some of the autoregulation data.

In any of the aspects or embodiments described above and herein, wherein the determination of the inflection point may include determining a second derivative of the curve.

In any of the aspects or embodiments described above and herein, the step of determining at least one of the LLA or the ULA of the subject's autoregulation function state using the fitted curve may include utilizing the first derivative of the fitted curve, the second derivative of the fitted curve, or the absolute value of the fitted curve, or any combination thereof.

In any of the aspects or embodiments described above and herein, wherein the step of determining at least one of the LLA or the ULA of the subject's autoregulation function state using the fitted curve may utilize an elbow point method.

In any of the aspects or embodiments described above and herein, wherein the step of determining autoregulation data as a function of subject blood pressure may include binning the autoregulation data determined for the period of time as a function of a plurality of incremental blood pressure bins, and the method may further include determining a confidence value for the autoregulation data in each incremental blood pressure bin.

In any of the aspects or embodiments described above and herein, wherein the step of fitting the curve to the autoregulation data may include evaluating the autoregulation data in each incremental blood pressure bin for inclusion in the curve fitting based on the determined confidence value for the autoregulation data in that respective incremental blood pressure bin.

In any of the aspects or embodiments described above and herein, wherein the step of determining said confidence value for the autoregulation data in each incremental blood pressure bin includes determining first order statistical information for the autoregulation data in each respective incremental blood pressure bin and determining the respective confidence value using the determined first order statistical information.

In any of the aspects or embodiments described above and herein, the method may further include visually displaying the binned autoregulation data as a function of the incremental blood pressure bins.

In any of the aspects or embodiments described above and herein, the method may further include visually displaying a confidence value for the autoregulation data in each incremental blood pressure bin with the binned autoregulation data.

According to an aspect of the present disclosure, an apparatus for determining a subject's autoregulation function state is provided that includes a near infra-red spectroscopy (NIRS) tissue oximeter, a blood pressure sensing device, and a controller. The NIRS tissue oximeter is configured to continuously sense a tissue region of the subject during a period of time, and to produce first signals representative of at least one tissue oxygenation parameter during the period of time. The blood pressure sensing device is configured to continuously measure a blood pressure level of the subject during the period of time, and to produce second signals representative of the blood pressure of the subject during the period of time. The controller is in communication with the NIRS tissue oximeter and the blood pressure sensing device. The controller includes at least one processor and a memory device configured to store instructions, the stored instructions when executed cause the controller to:

a) control the NIRS tissue oximeter to continuously sense a tissue region of the subject during a period of time, and to produce first signals representative of at least one tissue oxygenation parameter sensed within the tissue region during the period of time; b) control the blood pressure sensing device to continuously measure a blood pressure level of the subject during the period of time, and to produce second signals representative of the blood pressure of the subject during the period of time; c) determine autoregulation data using the first signals and the second signals; and and d) determine at least one of an LLA or a ULA of the subject's autoregulation function state.

In any of the aspects or embodiments described above and herein, the instructions when executed may include causing the controller to fit a curve to the autoregulation data as a function of subject blood pressure using an algorithm.

In any of the aspects or embodiments described above and herein, the instructions when executed may cause the controller to determine an algorithmic model of the curve and use the algorithmic model to fit the curve to the autoregulation data, and to determine an inflection point using the algorithmic model of the curve in the determination of the at least one of the LLA or ULA of the subject's autoregulation function state.

In any of the aspects or embodiments described above and herein, the instructions when executed that cause the controller to determine the autoregulation data as a function of subject blood pressure, may also cause the controller to bin the autoregulation data determined for the period of time as a function of a plurality of incremental blood pressure bins, and determine a confidence value for the autoregulation data in each incremental blood pressure bin.

In any of the aspects or embodiments described above and herein, the instructions when executed may cause the controller to evaluate the autoregulation data in each incremental blood pressure bin for inclusion in the curve fitting based on the determined confidence value for the autoregulation data in that respective incremental blood pressure bin.

According to an aspect of the present disclosure, one or more non-transitory computer-readable mediums may be provided comprising instructions for implementing one or more of the present disclosure embodiments described herein.

The foregoing features and elements may be combined in various combinations without exclusivity, unless expressly indicated otherwise. These features and elements as well as the operation thereof will become more apparent in light of the following description and the accompanying drawings. It should be understood, however, the following description and drawings are intended to be exemplary in nature and non-limiting.

BRIEF DESCRIPTION OF THE DRAWINGS

FIG. 1 is a diagrammatic illustration of autoregulation parameters as a function of time.

FIG. 2 is a diagrammatic illustration of blood flow versus perfusion pressure, indicating a relationship between dilated and constricted blood vessels and autoregulation function.

FIG. 3 is a diagrammatic illustration of cerebral blood flow versus cerebral perfusion pressure, indicating a normal condition, a hypotensive condition, and a hypertensive condition.

FIG. 4A is a diagrammatic representation of an autoregulation system according to an embodiment of the present disclosure.

FIG. 4B is a diagrammatic representation of an autoregulation system according to an embodiment of the present disclosure.

FIG. 5 is a diagrammatic representation of an exemplary frequency domain method.

FIG. 6 is an autoregulation profile plot embodiment example.

FIG. 7 is an autoregulation profile plot embodiment example.

FIG. 8 is an autoregulation profile plot embodiment example.

FIG. 9 is a functional diagram of an embodiment of an aspect of the present disclosure.

FIG. 10 is a diagram of multiple frequency bands shown in FIG. 9 depicted on a frequency axis.

FIG. 11 is a diagram of multiple frequency bands shown in FIG. 9 depicted on a time axis.

FIG. 12 is a diagrammatic display illustrating autoregulation plots for a plurality of NIRS indices.

FIG. 12A provides a diagrammatic example of the present disclosure embodiments that utilize a plurality of different NIRS indices.

FIG. 13 is a chart illustrating an exemplary relationship between phase and frequency.

FIG. 14 is a diagrammatic representation of an embodiment of an exemplary frequency domain method according to the present disclosure.

FIG. 15 is an exemplary display of autoregulation data according to an embodiment of the present disclosure.

FIG. 16 is an exemplary display of autoregulation data in the form of a sigmoidal curve according to an embodiment of the present disclosure.

FIG. 17 is an exemplary display of autoregulation data in the form of sigmoidal curves according to an embodiment of the present disclosure, on a plot of AR index versus mean blood pressure.

FIG. 18 is an exemplary display of autoregulation data in the form of sigmoidal curves according to an embodiment of the present disclosure, on a plot of AR index versus mean blood pressure.

FIG. 19 is an exemplary display of autoregulation data in the form of sigmoidal curves according to an embodiment of the present disclosure, on a plot of AR index versus mean blood pressure, including a plurality of autoregulation zones.

FIG. 20 is a graph illustrating an example of an elbow point methodology.

FIG. 21 is a diagrammatic illustration of a sigmoidal curve fit to CAI data as a function of binned blood pressure (MAP) values, where the LLA is determined using an elbow point technique.

FIG. 22 is a profile plot of CAI values versus binned blood pressure (MAP).

FIG. 23 is an exemplary display of autoregulation data in the form of a plot of AR index versus mean blood pressure, illustrating indicators of autoregulation data variance.

FIGS. 24A-24E are still shots taken from a video illustrating an autoregulation curve (AR Index values along the Y-axis and blood pressure values (binned) along the X-axis) production as a function of time, with FIGS. 24A-24E representing sequential points in time from the video.

FIG. 25 is an exemplary autoregulation curve showing a lower limit of autoregulation (LLA) determined from the curve fitted to the data.

FIG. 26 is a flow chart illustrating aspects of the present disclosure.

FIG. 27 is a functional diagram illustrating a confounding factor function embodiment.

FIG. 28 is a functional diagram illustrating a confounding factor function embodiment relating to physiological uncertainties.

FIG. 29 is an exemplary multiple trend input table including BP, $StO_2$ and THb trends.

FIG. 30 is a diagrammatic illustration of NIRS index trends subsequent to a physiological event.

FIG. 31 is a diagrammatic illustration of NIRS index trends subsequent to a physiological event.

FIG. 32 is a diagrammatic illustration of NIRS index trends subsequent to a physiological event.

FIG. 33 is a diagrammatic illustration of NIRS index trends subsequent to a physiological event.

FIG. 34 is a diagrammatic illustration of NIRS index trends subsequent to a physiological event.

FIG. 35 is a functional diagram illustrating a confounding factor function embodiment relating to hypoxia.

FIGS. 36-38 are functional diagrams illustrating confounding factor function embodiments relating to pain.

FIG. 39A is a graph of cerebral blood flow versus cerebral perfusion pressure illustrating the effects of hypercapnia.

FIG. 39B is a graph of cerebral blood flow versus cerebral perfusion pressure illustrating the effects of hypocapnia.

FIG. 40 is a functional diagram illustrating a confounding factor function embodiment relating to carbon dioxide levels within blood.

FIG. 41 is a functional diagram illustrating a confounding factor function embodiment relating to carbon dioxide levels within blood.

FIG. 42 is a functional diagram illustrating a confounding factor function embodiment relating to extracerebral blood flow.

DETAILED DESCRIPTION

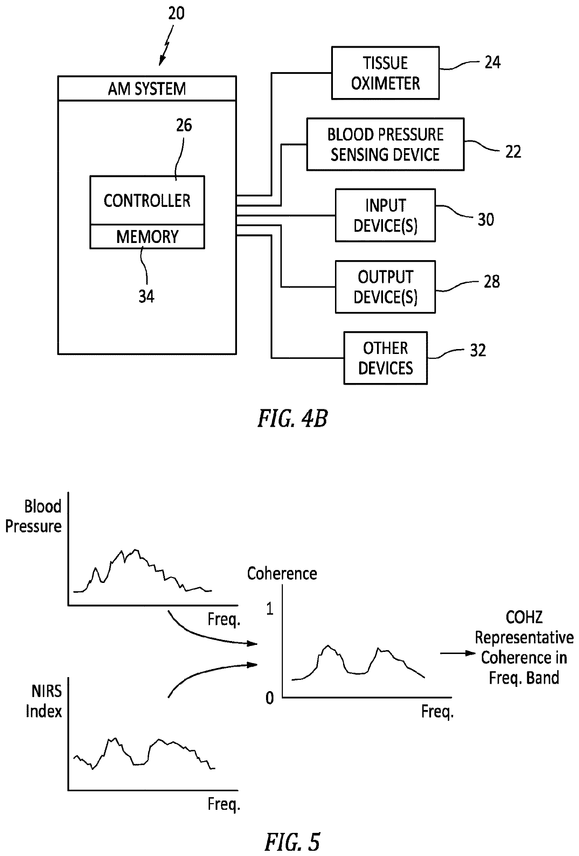

Referring to FIGS. 4A and 4B, a non-limiting embodiment of an autoregulation measurement and monitoring system ("AM system 20") is shown. As will be described herein, the AM system 20 may be configured to produce a data value (e.g., a coherence value) that can be measured and/or monitored, or a data value that is indicative of the state of a subject's autoregulation system function; e.g., the degree to which the subject's autoregulation system is functioning, or any combination thereof. While an exemplary AM system 20 is shown, the exemplary components illustrated in FIGS. 4A and 4B are not intended to be limiting; e.g., additional, or alternative components and/or implementations may be used. In some embodiments (e.g., FIG. 4A), the AM system 20 may include a blood pressure sensing device 22, a tissue oximeter 24, other devices 32 (e.g., a carbon dioxide ($CO_2$) sensor such as a transcutaneous blood gas monitor, or an exhaled bread $CO_2$ sensor, or the like, or combinations thereof, a heart rate monitor such as an electrocardiogram - "ECG", etc.), a controller 26, one or more output devices 28, and one or more input devices 30 integrated into a single system device; e.g., a controller 26 integrally connected with sensing hardware (e.g., hardware associated with a tissue oximeter, hardware associated with a blood pressure sensor, etc.). In other embodiments (e.g., FIG. 4B), the AM system 20 may include a controller 26, and may be configured to communicate with (e.g., receive signal data from and/or send signal data to) a blood pressure sensing device 22, a tissue oximeter 24, a $CO_2$ sensor, a heart rate monitor, one or more input devices 30, and one or more output devices 28. In other words, in these embodiments the AM system 20 may be configured to communicate with a blood pressure sensing device 22 that is capable of functioning independently of the AM system 20, a tissue oximeter 24 that is capable of functioning independently of the AM system 20, a $CO_2$ sensor that is capable of functioning independently of the AM system 20, a heart rate monitor that is capable of functioning independently of the AM system 20, etc. In other embodiments, the AM system 20 may include some combination of these devices in integral and independent form.

The blood pressure sensing device 22 ("BP sensing device 22") may be any sensor or device configured to continuously determine a subject's blood pressure (e.g., arterial blood pressure). For example, the BP sensing device 22 may a device that is configured to provide continuous blood pressure measurement, such as an arterial catheter line, or a continuous non-invasive blood pressure device, or a pulse oximetry sensor. The present disclosure is not, however, limited to using these particular examples of blood pressure sensing / measuring / monitoring devices 22. The BP sensing device 22 is configured to produce blood pressure value signals indicative of the subject's blood pressure (e.g., arterial blood pressure) during a period of time. The BP sensing device 22 is configured for communication with the AM system controller 26; e.g., send blood pressure value signals to the AM system controller 26, and may receive control signals, etc. from the AM system controller 26. Communications between the BP sensing device 22 and the AM system controller 26 may be by any known means; e.g., hardwire, wireless, etc. The term "continuously" as used herein (to describe a BP sensing device 22 continuously determining a subject's blood pressure) means that the BP sensing device 22 senses and collects subject data on a periodic basis during the monitoring time period, which periodic basis is sufficiently frequent that it may be considered to be clinically continuous. For example, some BP sensing devices 22 sample data every ten seconds or less and can be configured to sample data more frequently (e.g., every two seconds or less).

The tissue oximeter 24 may be a device configured to continuously sense a tissue oxygenation parameter (referred to hereinafter individually as a "NIRS index" or collectively as "NIRS indices") that varies with blood flow in a subject's tissue; e.g., tissue oxygen saturation (StO$_2$), total hemoglobin concentration per volume of tissue (THb), relative total hemoglobin concentration per volume of tissue (rTHb), deoxyhemoglobin (HHb), relative deoxyhemoglobin (rHHb), oxyhemoglobin (O2Hb), relative oxyhemoglobin (rO2Hb), differential changes in oxyhemoglobin (O2Hb) and deoxyhemoglobin (HHb), differential changes in relative oxyhemoglobin (rO2Hb) and deoxyhemoglobin (rHHb), HbD (i.e., O2Hb - HHb), etc. To be clear, the present disclosure is not limited to these particular NIRS indices and the various acronyms used herein (e.g., StO$_2$, THb, rTHb, HHb, rHHb, O2Hb, rO2Hb, and HbD) are non-limited examples of acronyms that may be used by those skilled in the art to refer to the respective NIRS index. A person of skill in the art will recognize that the same NIRS indices are sometimes referred to using different acronyms; e.g., Hb, HbO$_2$, TotalHb, etc. An example of an acceptable tissue oximeter 24 is a near infra-red spectroscopy ("NIRS") type tissue oximeter ("NIRS tissue oximeter"). U.S. Patent Nos. 6,456,862; 7,072,701; 8,078,250; 8,396,526; and 8,965,472; and 10,117,610, each of which is hereby incorporated by reference in its entirety, disclose non-limiting examples of a non-invasive NIRS tissue oximeter that may be used within the present disclosure. The term "continuously" as used herein (to describe a tissue oximeter 24 continuously sensing a tissue oxygenation parameter) means that the tissue oximeter 24 senses and collects subject data on a periodic basis during the monitoring time period, which periodic basis is sufficiently frequent that it may be considered to be clinically continuous. For example, some tissue oximeters 24 sample data every ten seconds or less and can be configured to sample data more frequently (e.g., every two seconds or less).

The tissue oximeter 24 includes one or more sensors in communication with a controller portion. Each sensor includes one or more light sources (e.g., light emitting diodes, or "LEDs") and one or more light detectors (e.g., photodiodes, etc.). The light sources are configured to emit light at different wavelengths of light, e.g., wavelengths of light in the red or near infrared range; 400-1000nm. In some sensor embodiments, a sensor may be configured to include a light source, a near detector(s), and a far detector(s). The near detector(s) are disposed closer to the light source than the far detector(s). A non-limiting example of such a sensor is disclosed in U.S. Patent No. 8,965,472, which as indicated above, is incorporated by reference in its entirety. The tissue oximeter 24 is configured for communication with the AM system controller 26; e.g., send signals representative of (or signals that can be used to determine) one or more NIRS indices to the AM system controller 26, and may receive control signals, etc. from the AM system controller 26. Communications between the tissue oximeter 24 and the AM system controller 26 may be by any known means; e.g., hardwire, wireless, etc.

The NIRS tissue oximeter 24 may utilize one or more algorithms for determining one or more of the NIRS indices. The present disclosure is not limited to any particular NIRS tissue oximeter 24 or any algorithm for determining a NIRS Index of the sensed tissue. U.S. Patent Nos. 9,913,601; 9,848,808; 9,456,773; 9,364,175; 9,923,943; 8,788,004; 8,396,526; 8,078,250; 7,072,701; and 6,456,862 all describe non-limiting examples of algorithms for determining NIRS indices that may be used with the present disclosure, and all are incorporated by reference in their respective entirety herein.

One or both of the BP sensing device 22 or the tissue oximeter 24 may be further configured to measure other parameters, such as respiratory rate, respiratory effort, heart rate, etc. The BP sensing device 22 and the tissue oximeter 24 may be placed on the same or different parts of the patient's body.

As stated above, the BP sensing device 22, the tissue oximeter 24, and other devices identified herein may be integrated within the AM system 20, or they may be independent devices that provide signal data to the AM system 20, or any combination thereof. In those embodiments wherein one or more of the aforesaid devices is independent of the AM system 20, that independent device may be in communication with the AM system controller 26 in any manner.

As stated above, the AM system 20 includes a controller 26, and may include one or more output devices 28 and one or more input devices 30. Non-limiting examples of an input device 30 include a keyboard, a touchpad, or other device wherein a user may input data, commands, or signal information, or a port configured for communication with an external input device via hardwire or wireless connection, etc. Non-limiting examples of an output device 28 include any type of display, printer, or other device configured to display or communicate information or data produced by the AM system 20. The AM system 20 may be configured for connection with an input device 30 or an output device 28 via a hardwire connection or a wireless connection.

In some embodiments, the AM system controller 26 may be configured (e.g., via electrical circuitry) to process various received signals (received from integral or independent devices) and may be configured to produce certain signals to the same; e.g., signals configured to control one or more components within the AM system 20. Alternatively, the AM system 20 may be configured such that signals from the respective component are sent to one or more intermediate processing devices, and the intermediate processing device may in turn provide processed signals or data to the AM system controller 26. As will be explained below, the AM system controller 26 may also be configured to execute stored instructions (e.g., algorithmic instructions) that cause the AM system 20 to perform steps or functions described herein, to produce data (e.g., measurements, etc.) relating to a subject's autoregulation system, to communicate, etc.

The AM system controller 26 may include any type of computing device, computational circuit, or any type of process or processing circuit capable of executing a series of instructions that are stored in memory 34. The controller 26 may include multiple processors and/or multicore CPUs and may include any type of processor, such as a microprocessor, digital signal processor, co-processors, a micro-controller, a microcomputer, a central processing unit, a field programmable gate array, a programmable logic device, a state machine, logic circuitry, analog circuitry, digital circuitry, etc., and any combination thereof. For example, in those embodiments of the AM system 20 described above that include multiple components integral with the system 20 (e.g., a blood pressure sensing device 22, a tissue oximeter 24, a $CO_2$ sensor, etc.) integral with the system, the controller 26 may include multiple processors; e.g., an independent processor dedicated to each respective component, any and all of which processors may be in communication with a central processor of the AM system 20 that coordinates functionality of the controller 26 / AM system 20. The instructions stored in memory may represent one or more algorithms for controlling the AM system 20, and the stored instructions are not limited to any particular form (e.g., program files, system data, buffers, drivers, utilities, system programs, etc.) provided they can be executed by the controller 26. The instructions are configured to perform the methods and functions described herein.

The memory 34 may be a machine readable storage medium configured to store instructions that when executed by one or more processors, cause the one or more processors to perform or cause the performance of certain functions. The memory 34 may be a single memory device or a plurality of memory devices. A memory device may be a non-transitory device and may include a storage area network, network attached storage, as well as a disk drive, a read-only memory, random access memory, volatile memory, non-volatile memory, static memory, dynamic memory, flash memory, cache memory, and/or any device that stores digital information. One skilled in the art will appreciate, based on a review of this disclosure, that the implementation of the controller 26 may be achieved via the use of hardware, software, firmware, or any combination thereof.

Implementation of the techniques, blocks, steps, and means described herein may be done in various ways. For example, these techniques, blocks, steps, and means may be implemented in hardware, software, or a combination thereof. For a hardware implementation, processing devices configured to carry out the described functions and steps (e.g., by executing stored instructions) may be implemented within one or more application specific integrated circuits (ASICs), digital signal processors (DSPs), digital signal processing devices (DSPDs), programmable logic devices (PLDs), field programmable gate arrays (FPGAs), processors, controllers, micro-controllers, microprocessors, or other electronic units designed to perform the functions described herein, and/or any combination thereof.

Also, it is noted that the embodiments of the present disclosure may be described herein as a process which is depicted as a flowchart, a flow diagram, a block diagram, etc. Although any one of these structures may describe the operations as a sequential process, many of the operations can be performed in parallel or concurrently. In addition, the order of the operations may be rearranged. A process may correspond to a method, a function, a procedure, a subroutine, a subprogram, etc.

The present AM system 20 utilizes real-time data collection of tissue oximeter 24 data (e.g., relating to one or more NIRS indices) and continuous blood pressure measurement data to produce data relating to a subject's autoregulation function. The specific functionality of the tissue oximeter 24 and the BP sensing device 22 (e.g., sampling rate, etc.) can be set as appropriate for the operation of the AR system 20, and the present disclosure is not limited to any particular device settings. The tissue oximeter 24 data and the BP sensing device 22 data (e.g., in signal form) are sent to the AR system controller 26 where they are processed using stored instructions to determine autoregulation data. For example, the present AM system 20 may be configured to produce data indicative of a correlation between at least one NIRS Index and blood pressure data to determine autoregulation data for a subject. In some embodiments, the AM system 20 may be configured to use an algorithm based on a frequency domain methodology to produce a coherence analysis.

In those embodiments of the present AM system 20 that include a $CO_2$ sensor (e.g., a transcutaneous blood gas monitor, an exhaled breadth $CO_2$ sensor, or the like, or any combination thereof), the AM system 20 may utilize real-time data collection of $CO_2$ sensor data. The specific functionality of the $CO_2$ sensor (e.g., sampling rate, etc.) can be set as appropriate for the operation of the AR system 20, and the present disclosure is not limited to any particular device settings. The $CO_2$ sensor data (e.g., in signal form) are sent to the AR system controller 26 where they are processed using stored instructions as described herein.

FIG. 5 diagrammatically depicts an exemplary frequency domain method that involves taking synchronous blood pressure and NIRS index values over a predetermined sampling window (e.g., period of time). As stated herein, aspects of the present disclosure are not limited to using a frequency domain method. In this exemplary frequency domain method, the blood pressure and NIRS index values are each transformed (e.g., via a Fourier transformation) from a time domain to a frequency domain (shown as respective plots of blood pressure versus frequency and NIRS Index versus frequency; the transformed tissue oxygenation parameter values (e.g., the NIRS index) may be referred to as a "frequency domain tissue oxygenation parameter values", and the transformed blood pressure values may be referred to as "frequency domain blood pressure values"), and the transformed data is further analyzed to determine the degree of coherence within a single band of frequencies (i.e., a single frequency band). The degree of coherence may be indicated in terms of an arbitrarily assigned scale of zero to one (0 - 1), wherein the degree of coherence increases from zero to one (shown as a plot of coherence values versus frequency). A coherence value of one represents a pressure passive condition as described above. Conversely, a coherence value that approaches zero indicates increasingly less relationship between the NIRS index and blood pressure parameters. A coherence value ("COHZ") that is representative of substantially all frequencies in the single frequency band may be used as an autoregulation ("AR Index") or pressure passive index ("PPI"). For purposes of the present description, the terms "AR Index" and pressure passive index "PPI" are intended to be substantially equivalent, and for sake of clarity the term "AR Index" will be used hereinafter. The representative coherence value ("COHZ") for a single frequency band may be an average of the coherence values within the single frequency band, or a mean value, or a median value, or any similar value that collectively represents the coherence values over all frequencies in the single frequency band.

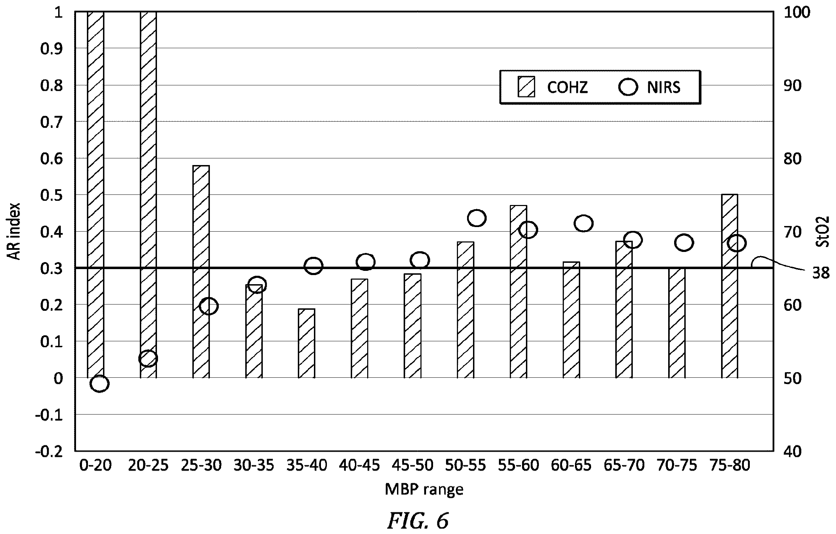

In some embodiments, the COHZ values (within the single frequency band) determined over a period of time may be binned in blood pressure increments (e.g., every 5 mmHg) or in incremental blood pressure ranges (e.g., 0-20 mmHg, 20-25 mmHg, 25-30 mmHg, etc.). Non-limiting examples of autoregulation profile plots over a few hours are shown in FIGS. 6-8, which autoregulation profile plots are based on COHZ values determined within a single frequency band.

In FIG. 6, an autoregulation profile plot based on pig lab data is shown, depicting Y-axes of an AR Index and a representative StO$_2$ value (i.e., a NIRS index), an X-axis of a representative blood pressure range (shown in 5 mmHg bins), and coherence values ("COHZ") per blood pressure bin. The representative StO$_2$ value may be a mean value, an average value, a median value, or similar value that collectively represents the StO$_2$ values over all frequencies in the band. In alternative ambodiments, the autoregulation profile plot may include a NIRS index other than StOz; i.e., THb, rTHb, differential changes in O2Hb and HHb, HbD, etc. As can be seen in FIG. 6, the COHZ values may be viewed in terms of the AR Index. The data depicted in FIG. 6 indicates that the autoregulation of the subject pig becomes increasingly pressure passive at a blood pressure value less than about thirty mmHg (30 mmHg). FIG. 6 includes a horizontal line 38 at about the AR Index value of 0.3 to reflect an AR Index value inflection point above which the subject's autoregulation system may be described as being pressure passive to some degree (e.g., the degree to which the subject's autoregulation system is pressure passive increases as the AR Index approaches an AR Index value of 1), and below which the subject's autoregulation function is substantially normal. The present disclosure is not limited to the AR Index value inflection point of 0.3, or to any particular AR Index value inflection point. The AR Index value inflection point may be based on empirical data, and may vary depending on factors such as characteristics of the subject; e.g., age, health, smoker, etc.

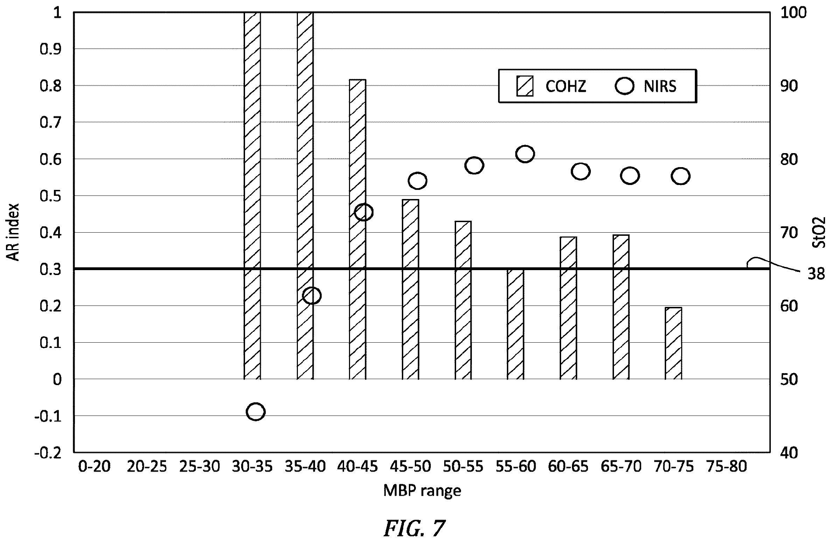

In FIG. 7, an autoregulation profile plot based on human neonate data is shown, depicting Y-axes of an AR Index and a representative StO$_2$ (i.e., a NIRS Index), an X-axis of a representative blood pressure range (shown in 5 mmHg bins), and coherence values ("COHZ") per blood pressure bin. As stated above, the autoregulation profile plot may include a NIRS Index other than StO$_2$; i.e., rTHb, differential changes in O2Hb and HHb, HbD, etc. The data depicted in FIG. 7 indicates that the autoregulation of the human neonate subject becomes increasingly pressure passive at a blood pressure value less than about fifty mmHg (50 mmHg). FIG. 7 includes a horizontal line 38 at about the AR Index value of 0.3 to reflect an AR Index value inflection point above which the subject's autoregulation system may be described as being pressure passive to some degree, and below which the subject's autoregulation function is substantially normal. As stated above, the present disclosure is not limited to the AR Index value inflection point of 0.3, or to any particular AR Index value inflection point.

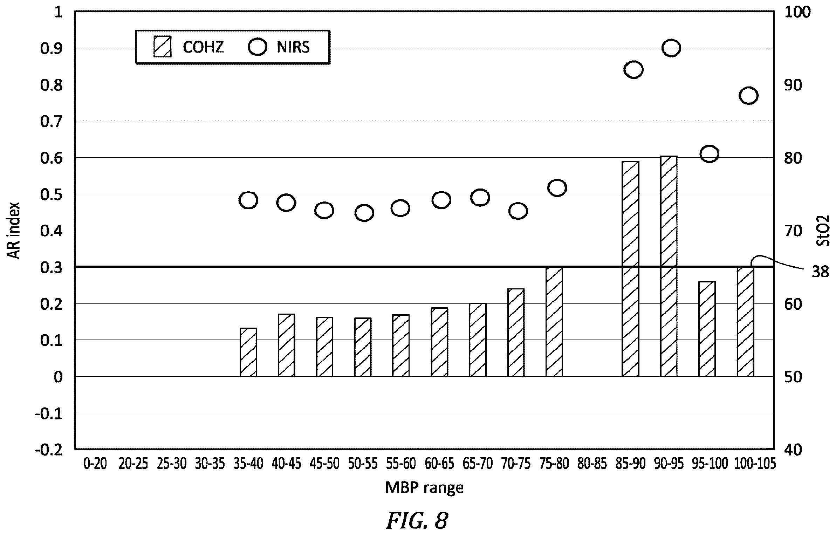

In FIG. 8, an autoregulation profile plot based on human neonate data is shown, depicting Y-axes of an AR Index and a representative StO$_2$ (i.e., a NIRS Index), an X-axis of a representative blood pressure range (shown in 5 mmHg bins), and coherence values ("COHZ") per blood pressure bin. As stated above, the autoregulation profile plot may include a NIRS index other than StO$_2$; i.e., rTHb, differential changes in O2Hb and HHb, HbD, etc. The data depicted in FIG. 8 indicates that the autoregulation of the human neonate subject becomes increasingly pressure passive at a blood pressure value greater than about eighty-five mmHg (85 mmHg). FIG. 8 includes a horizontal line 38 at about the AR Index value of 0.3 to reflect an AR Index value inflection point above which the subject's autoregulation system may be described as being pressure passive to some degree, and below which the subject's autoregulation function is substantially normal. As stated above, the present disclosure is not limited to the AR Index value inflection point of 0.3, or to any particular AR Index value inflection point.

Aspects of the present disclosure may provide enhanced measurement of a subject's autoregulation function (e.g., the degree to which a subject's autoregulation system is functioning), or an enhanced determination of the state of the subject's autoregulation function, or any combination thereof. For example, in some embodiments the present disclosure includes determining and analyzing COHZ values from different predetermined frequency bands simultaneously (or nearly simultaneously) from NIRS tissue oximetry and physiological (e.g., mean blood pressure) data taken from different sampling windows, and determining a peak COHZ value (i.e., a "MAX COHZ" value) at a given point in time from the COHZ values determined within the different predetermined frequency bands. The MAX COHZ value may be determined periodically (e.g., every 30 seconds). In this way, the MAX COHZ value used for further analysis could be based on the COHZ value determined from any of the different predetermined frequency bands; e.g., at a first point in time the MAX COHZ value may be based on data from a first frequency band, and at another point in time the MAX COHZ value may be based on data from a different frequency band, etc. As will be explained below, the possibility of determining a MAX COHZ value from a plurality of different predetermined frequency bands, as opposed to it being determined from a single frequency band, is believed to increase the sensitivity and accuracy of the AM system 20, and to improve the real-time response detection of the AM system 20 (e.g., improve the ability of the AM system 20 to detect an issue more rapidly with a subject's autoregulation function).

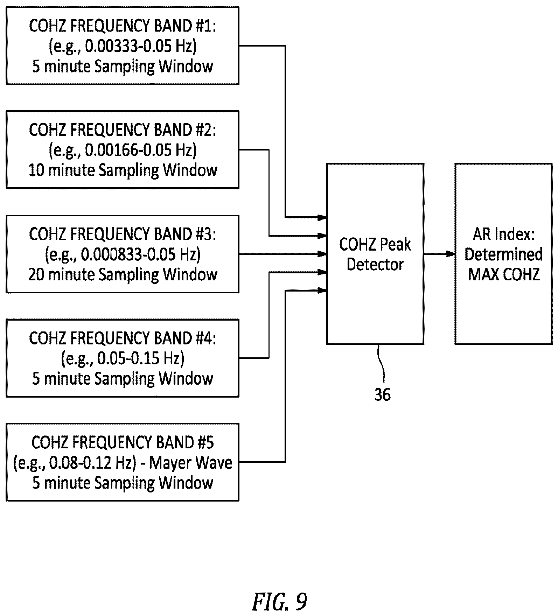

Referring to FIG. 9, a representative coherence value ("COHZ") may be determined in at least a plurality of the predetermined frequency bands (e.g., in a method similar to that described above with respect to FIG. 5), and a real-time peak coherence value (MAX COHZ) may be determined from those COHZ values (i.e., determined from the COHZ values determined in a respective different frequency band). For example, in the exemplary methodology shown in FIG. 9, there are five different frequency bands shown. Frequency band number 1 ("#1") has a bandwidth of 0.00333 Hz to 0.05 Hz and a five minute sampling window. Frequency band number 2 ("#2") has a bandwidth of 0.00166 Hz to 0.05 Hz and a ten minute sampling window. Frequency band number 3 ("#3") has a bandwidth of 0.000833 Hz to 0.05 Hz and a twenty minute sampling window. Hence, frequency band numbers #1-3 represent different bandwidths and different sampling windows; e.g., the range of frequencies within frequency band numbers #1-3 are chosen at least in part based on the duration of the associated sampling window; e.g., 5 mins, 10 mins, 20 mins, etc. The range of frequencies within a frequency band may also be chosen in view of the sampling rate of the tissue oximeter 24, or the sampling rate (or collection rate) of the blood pressure sensing device 22, or both or some combination thereof; e.g., the frequency band may be chosen such that the sampling rate of the respective device is within the frequency band. Frequency band #1 is understood to be effective for identifying coherence (e.g., a coherence that is readily identifiable) when there is a rapid change in a subject's blood pressure. Frequency band #2 is understood to be effective for identifying coherence (e.g., a coherence that is readily identifiable) when changes in a subject's blood pressure are less rapid than those considered within frequency band #1. Frequency band #3 is understood to be effective for identifying coherence (e.g., a coherence that is readily identifiable) when changes in a subject's blood pressure are less rapid than those considered within frequency band #2. The frequency ranges for frequency bands #1-3 described above are examples, and the present disclosure is not limited to these particular frequency ranges. Frequency band number #4 has a bandwidth of 0.05 Hz to 0.15 Hz and a five minute sampling window. The range of frequencies within frequency band #4 is chosen to permit evaluation of a range of frequencies higher than those within frequency band #1-3 and is understood to be effective for identifying coherence (e.g., a coherence that is readily identifiable) when there is a rapid change in a subject's blood pressure, and/or may be chosen to reflect respiratory effects (e.g., breathing rate, etc.). The frequency range for frequency band #4 described above is also an example, and the present disclosure is not limited to this particular frequency range. Frequency band number #5 has a bandwidth of 0.08 Hz to 0.12 Hz and a five minute sampling window. The range of frequencies within frequency band #5 may be chosen to evaluate physiological characteristics of the subject (e.g., Mayer waves), and is understood to be effective for identifying coherence (e.g., a coherence that is readily identifiable) associated with Mayer waves. Mayer waves are cyclic changes (e.g., "waves") in arterial blood pressure brought about by oscillations in baroreceptor and chemoreceptor reflex control systems. Mayer waves may be defined as arterial blood pressure oscillations at frequencies slower than respiratory frequency and which show the strongest, significant coherence (strength of linear coupling between fluctuations of two variables in the frequency domain) with efferent sympathetic nervous activity. The frequency range for frequency band #5 is also an example, and the present disclosure is not limited to this particular frequency range.

Embodiments of the present disclosure that determine a MAX COHZ from a plurality of predetermined frequency bands are not limited to the above disclosed frequency bands or the identified sampling windows; e.g., fewer or more bands associated with different duration sampling windows may be used, and/or different sampling windows may be used, etc. The above-disclosed frequency bands and sampling windows are understood to provide considerable utility as will be described below, but the present disclosure is not limited thereto.

By determining COHZ values within a plurality of predefined frequency bands (e.g., like those shown in FIG. 9), the highest COHZ value (i.e., the MAX COHZ value) can be selected from the different frequency bands via a COHZ peak detector 36 at any given point in time (e.g., including periodic determinations as indicated herein). The MAX COHZ value provides greater sensitivity to autoregulation function at any given point in time as compared to a COHZ value determined from a single frequency band; e.g., as shown in methodology depicted in FIG. 5. As a result, the MAX COHZ value (and corresponding AR Index) is more indicative of the real-time (present time) circumstances and the clinician can be alerted more rapidly especially if the subject's blood pressure falls below the lower autoregulation threshold (e.g., the "LLA", which in a graph solution appears as a lower blood pressure deflection point). For example, if there is a rapid change in a subject's blood pressure and in a NIRS index (e.g., $StO_2$), the COHZ value determined from a higher frequency band will likely be substantially higher than the COHZ value determined from a lower frequency band. Hence, the "event" (i.e., the rapid change in a subject's blood pressure and in a NIRS index) is more rapidly identified within the higher frequency band. Conversely if there is a slow simultaneous change in a subject's blood pressure and in a NIRS index (e.g., $StO_2$), the COHZ value determined from a lower frequency band will likely be substantially higher than the COHZ value determined from a higher frequency band. Hence, the "event" (i.e., the slow change in a subject's blood pressure and in a NIRS index) is more rapidly identified within the lower frequency band.

There is significant clinical value in determining an indication of change in a subject's autoregulation functioning (e.g., if the autoregulation function is failing, such as a pressure passive condition, etc.) as quickly as possible. Autoregulation monitoring systems that monitor a subject's autoregulation functioning via a frequency domain approach that utilizes a single frequency band may be slower to report a high coherence value, or the magnitude of a coherence value may be diluted by lower coherence values at lower frequencies due to the averaging of all individual frequency coherence values. Embodiments of the present disclosure mitigate these limitations by determining COHZ values within a plurality of predefined frequency bands and determining a MAX COHZ value therefrom.

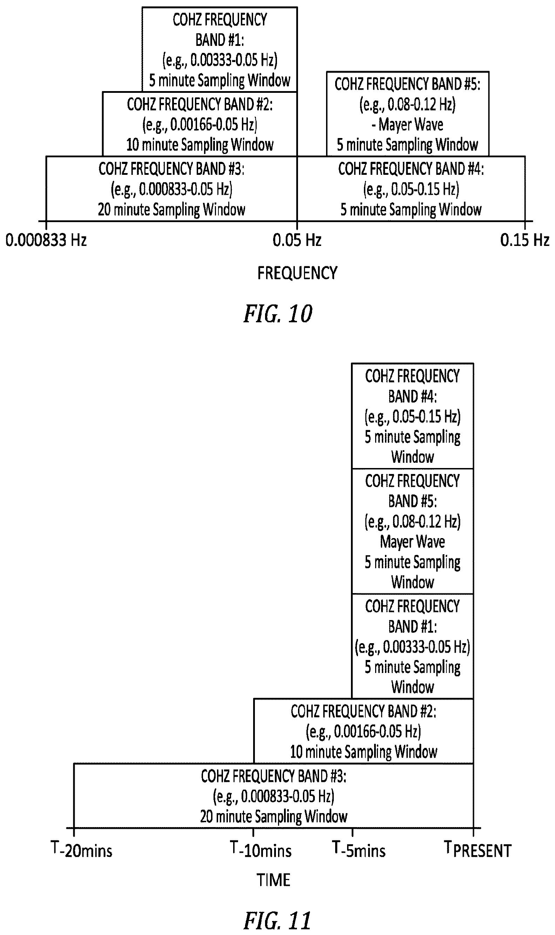

The diagrammatic illustration shown in FIG. 10 depicts a frequency domain methodology such as that shown in FIG. 9 and described above. In FIG. 10, the predetermined frequency bands #1-5 are shown on a horizontal frequency axis to illustrate the differences in the respective frequency bands.

The diagrammatic illustration shown in FIG. 11 shows time domain sampling windows corresponding to the exemplary predetermined frequency bands #1-5 shown in FIG. 9 and described above. The orientation of the time domain sampling windows shown in FIG. 11 illustrates that in some embodiments of the present disclosure, the autoregulation data produced at a given point in time ("$T_{Present}$") may be based on the time sampling windows representative of the immediate past 5 minutes ("$T_{-5mins}$"), 10 minutes ("$T_{-10mins}$"), and 20 minutes ("$T_{-20mins}$"); i.e., sampling windows that coincide at least partially. As stated above, the present disclosure is not limited to these particular sampling window durations.

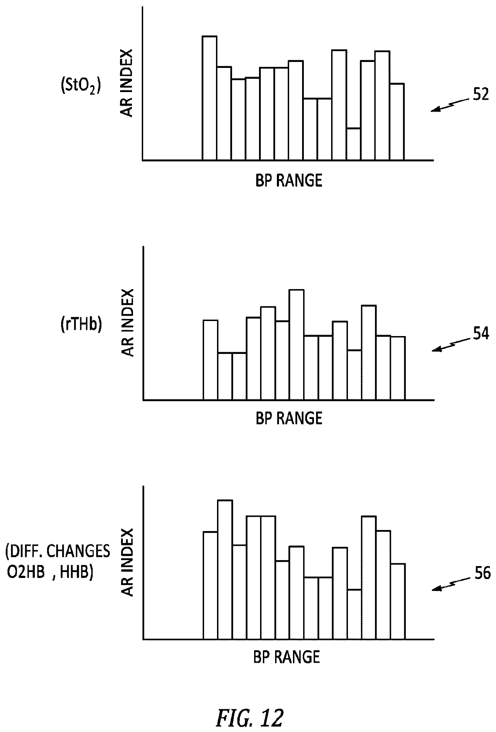

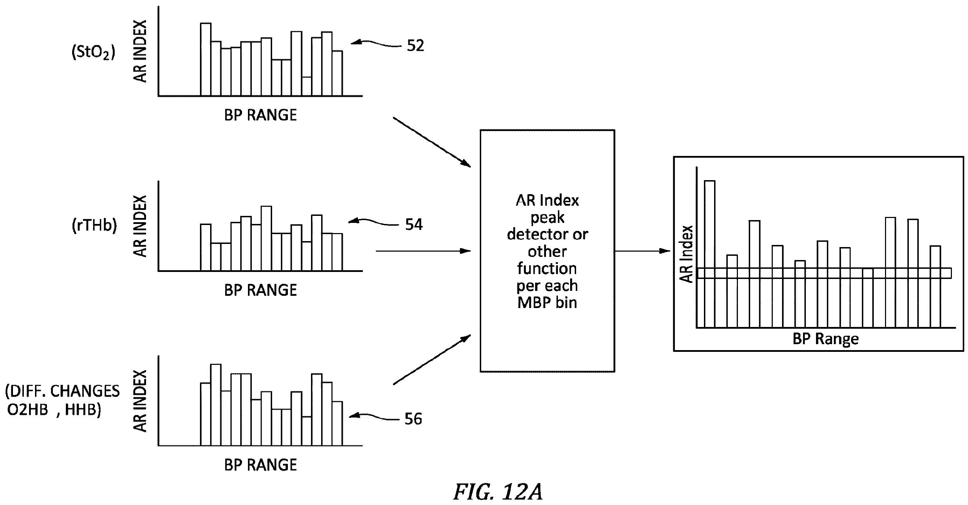

Other aspects of the present disclosure may also provide enhance measurement of a subject's autoregulation function. As described above, a subject's autoregulation functioning may be evaluated using synchronous blood pressure and NIRS index values over a period of time, where the blood pressure and NIRS index values are each transformed from a time domain to a frequency domain, and the transformed data is further analyzed to determine the degree of coherence there between. In some embodiments of the present disclosure, this process may be executed for a plurality of different NIRS indices (e.g., executed using at least two of $StO_2$, rTHb, differential changes in O2Hb and HHb, HbD, etc.). In an instance where one NIRS index is more sensitive to autoregulation function than another, performing the autoregulation function determination processes as described herein (e.g., within a single frequency band, or within a plurality of frequency bands) can provide additional sensitivity and/or faster identification of change in a subject's autoregulation function. FIG. 12, for example, shows a first autoregulation plot 52 (AR Index v. BP Range) based on a first NIRS index (e.g., $StO_2$), a second autoregulation plot 54 based on a second NIRS index (e.g., rTHb), and a third autoregulation plot 56 based on a third NIRS index (e.g., differential changes in O2Hb and HHb). The COHZ values for each of the aforesaid NIRS indices can be evaluated relative to one another on a per blood pressure bin basis; e.g., the COHZ value associated with $StO_2$ for the 50-55 mmHg bin, the COHZ value associated with THb for the 50-55 mmHg bin, and the COHZ value of the differential changes in $HbO_2$ and Hb for the 50-55 mmHg bin, etc. In some embodiments, the evaluation process may include choosing the NIRS index with the highest COHZ value for that bin. FIG. 12A provides a diagrammatic example of the above methodologies, as well as a diagrammatic view of how the aforesaid methodologies may be displayed. In other embodiments, the evaluation process may include creating an average COHZ value (or a mean or median value, etc.) based on the COHZ values for the aforesaid NIRS indices for that bin. In some instances, a first NIRS index value may be more sensitive to autoregulation function than another (or in other instances, one NIRS index may be affected by a physiological event, whereas another NIRS index is not affected - or less affected by the same physiological event) and performing the autoregulation function determination processes as described above can provide additional sensitivity and/or faster identification of change in a subject's autoregulation function. The present disclosure is not limited to any particular methodology for monitoring a subject's autoregulation functioning using a plurality of different NIRS indices. For example in a first embodiment, the methodologies described herein for determining a MAX COHZ value can be performed for each NIRS index, and the MAX COHZ values from each such determination (i.e., MAX $COHZ_{NIRS\ INDEX\ 1}$, MAX $COHZ_{NIRS\ INDEX\ 2}$, MAX $COHZ_{NIRS\ INDEX\ 3}$, etc.) may then be evaluated relative to one another to choose a maximum value therefrom (e.g., a MAX $COHZ_{NIRS\ INDICES}$) that may then be used to evaluate the subject's autoregulation function as described herein. As another example, the plurality of different NIRS indices may be utilized elsewhere (e.g., earlier) in the MAX COHZ value determination. For example, during the processes for determining a COHZ value for each frequency band, a COHZ value may be determined for each NIRS Index within a particular frequency band (e.g., for a first frequency band: $COHZ_{NIRS\ INDEX\ 1-FB1}$, $COHZ_{NIRS\ INDEX\ 2-FB1}$, $COHZ_{NIRS\ INDEX\ 3-FB1}$), and a peak COHZ value chosen therefrom, and the process repeated for each frequency band. A peak coherence value (MAX COHZ) may then be determined from the aforesaid COHZ values; e.g., in a manner described herein. These exemplary methodologies for monitoring a subject's autoregulation functioning using a plurality of different NIRS indices are meant to be illustrative and not limiting.

In some embodiments, once a MAX COHZ value is determined from the coherence values (COHZ) determined from a plurality of predetermined frequency ranges being analyzed at that moment of time, the MAX COHZ value may be binned in blood pressure ranges (e.g., every 5 mmHg); e.g., if a small change in blood pressure is detected. In some embodiments, MAX COHZ values may be continuously determined on a periodic basis (e.g., every 30 seconds) over a given period of time (e.g., hours) and those MAX COHZ values may be further processed, for example, to facilitate display of the information. For example, periodically determined MAX COHZ values collected over a period of time may be binned and a representative value of the binned values (e.g., an average, mean, or median value) may be displayed within an autoregulation profile plot; e.g., a plot structured similar to those shown in FIGS. 6-8. In those embodiments that include a binning process wherein a representative value is determined for each bin, the process of producing the representative value (e.g., determining an average, mean, or median value) may provide an additional advantage of mitigating outlier values (e.g., false positives and false negatives).

In order to enhance visibility of autoregulation data to a clinician (e.g., to make it easier to recognize poor autoregulation), some embodiments of the present disclosure may manipulate MAX COHZ values (e.g., by a multiplier, or by a mathematical function, etc.) to make changes in a subject's autoregulation function (e.g., MAX COHZ values) easier to recognize. For example, in some embodiments an autoregulation profile may include an AR Index based on a mathematical function such as the following:

$$AR\ Index = 2\ x\ (MAX\ COHZ)^2 \qquad [\text{Eqn. 1}]$$

In addition, as stated above, the visibility of autoregulation data to a clinician may be enhanced by displaying a line that reflects an AR Index value inflection point above which the subject's autoregulation system may functioning poorly (e.g., functioning in a pressure passive manner). The exemplary autoregulation plot profiles shown in FIGS. 6-8, and 15 include an AR Index value inflection line 38 at 0.3. The present disclosure is not limited to autoregulation plot profiles that include an AR Index value inflection line 38, and for those profile embodiments that do include an AR Index value inflection line 38, they are not limited to 0.3 or any other particular AR Index value.

In some embodiments of the present disclosure, an autoregulation profile plot may reflect data for an entire monitoring period. In some embodiments, an autoregulation profile plot may reflect data collected during a period of time less than the entire monitoring period. A present disclosure AR system may be configured to selectively display either of these embodiments.

In some embodiments, the AR system may be configured to permit a plurality of autoregulation profile plots to be displayed simultaneously (e.g., on the same display screen); e.g., a first autoregulation profile plot displaying data collected over a long period of time during the monitoring period, as well as a second autoregulation profile plot displaying data collected over a shorter period of time during the monitoring period; e.g., a more recent period of time.

A NIRS index change or a blood pressure change does not necessarily implicate a subject's autoregulation function. An autoregulation function is typically in response to related changes in a NIRS index and blood pressure. For example, if a NIRS index changes within a relatively short period of time (e.g., 30 seconds) of a blood pressure change, then COHZ values derived from NIRS index changes and blood pressure changes are likely attributable to the subject's physiology and represent a valid indicator of autoregulation function. Conversely, consider a NIRS index change that occurs a relatively long period of time (e.g., 2 minutes) after a blood pressure change. The temporal separation between these two events makes it less likely that they related to one another as a physiological response. Hence, COHZ values derived from these temporally distinct changes are less likely attributable to the subject's physiology and the COHZ values would likely be a poor indicator of autoregulation function. The temporally distinct changes are more likely attributable to other physiological events such as hypoxia or outside interference such as subject movement.

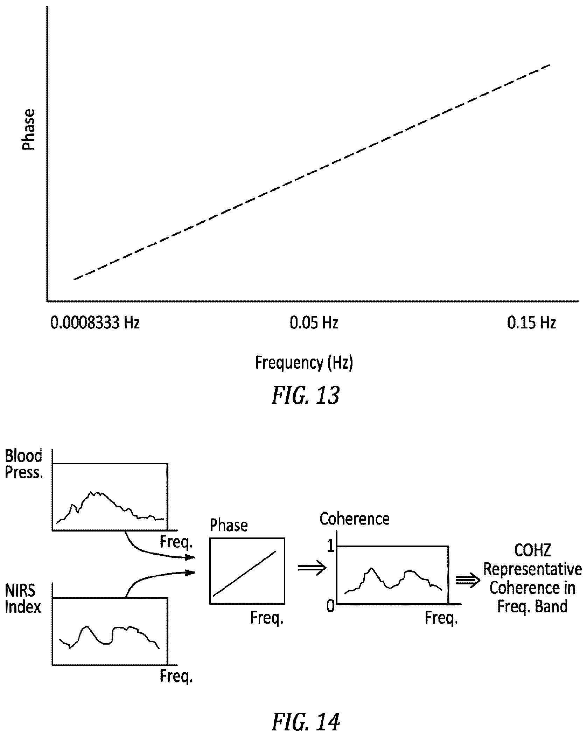

Referring to FIGS. 13 and 14, embodiments of the present disclosure consider a temporal relationship between NIRS index changes and blood pressure changes in evaluating a subject's autoregulation function. For example, in some embodiments, coherence values determined in particular frequency bands may be evaluated in terms of a "phase" range. The terms "phase" or "phase range" as used herein are used to mean a predetermined temporal relationship between the NIRS index change occurrence and the blood pressure change occurrence, or a frequency relationship between the NIRS index change occurrence and the blood pressure change. For example, a phase may be defined as:

$$\frac{Predetermined\ NIRS\ Index\ Response\ time\ to\ a\ change\ in\ Blood\ Pressure}{1/frequency} \qquad [\text{Eqn. 2}]$$

The above mathematical relationship is a non-limiting example of how the term "phase" may be defined, and the present disclosure is not limited to this particular mathematical relationship. In some embodiments, the phase relationship between the NIRS index change occurrence and the blood pressure change may be expressed in terms of the relationship between the aforesaid values expressed in a frequency domain, and the extent to which the aforesaid values in a frequency domain are out of phase with one another.

To illustrate how phase may be used to evaluate the validity of coherence values, consider coherence values determined within a particular frequency band (e.g., a very low frequency band). If the phase (e.g., the time separation between the change in blood pressure and the change in NIRS index) is outside of a predetermined phase range, then the respective determined coherence value can be discarded, or assigned a value (e.g., a low value such as zero) that will not corrupt the COHZ determination for that particular frequency band. The phase evaluation of an individual frequency may be performed before the coherence values for the particular frequency band are processed (e.g., averaged) to produce the COHZ value for that particular frequency band. As shown in FIG. 13, the maximum phase allowable as a function of NIRS response time to blood pressure change increases with frequency; e.g., at higher frequencies, all phase values may be physiologically valid when evaluating a subject's autoregulation function, whereas at very low frequencies only limited phase values may be physiologically valid (e.g., the temporal relationship between the blood pressure change and the NIRS Index change is too great and therefore less likely attributable to the subject's physiology) when evaluating a subject's autoregulation function.

In some instances, a subject may experience an acute blood pressure drop that may go below or above a lower autoregulation blood pressure range. In such instances, the present AR system may be configured (e.g., via stored algorithmic instructions) to update the displayed autoregulation information, including an autoregulation profile plot. The displayed information may include high values above a predetermined AR Index (or PPI Index) value indicative of a threshold autoregulation function (which value may be depicted as an AR Index value inflection line) above which the subject's autoregulation function becomes increasingly pressure passive.

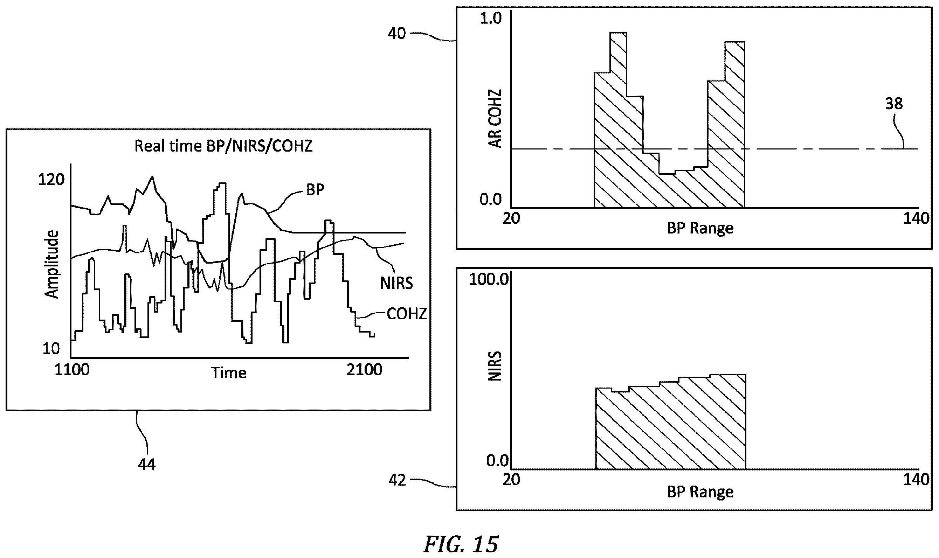

Some embodiments of the present disclosure may display one or more autoregulation plots, a short real-time window showing blood pressure and NIRS index signals and corresponding coherence signal. Some embodiments of the present disclosure may display binned values of a NIRS index as a function of blood pressure, similar to that of the autoregulation plot. The binning of a NIRS index value (e.g., a $StO_2$ value), may be triggered with at least a small change in blood pressure. A non-limiting example of a display embodiment is shown in FIG. 15, which depicts a display showing an autoregulation profile plot 40 (e.g., AR Index or COHZ versus BP Range), a corresponding plot 42 of binned NIRS index (e.g., $StO_2$) values versus BP Range, and a real-time window 44 showing blood pressure (e.g., a mean blood pressure), a NIRS index (e.g., $StO_2$), and COHZ as a function of time.

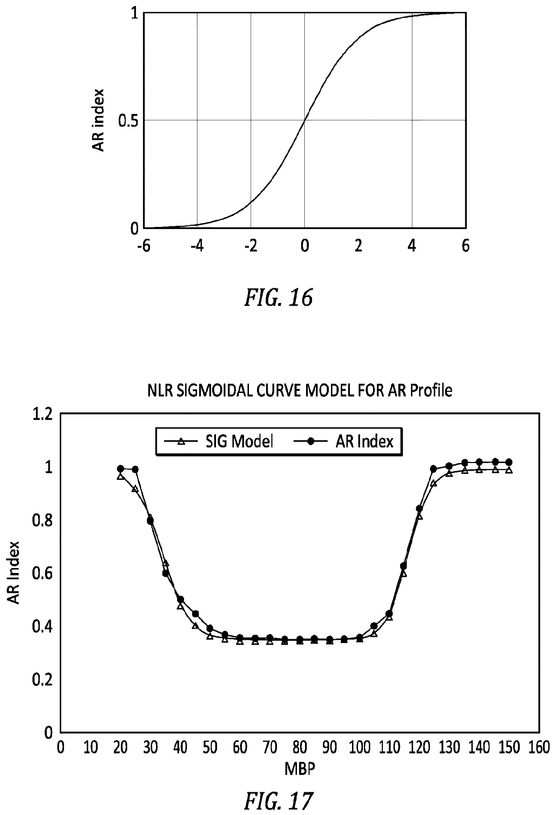

Autoregulation data produced according to present disclosure embodiments may be displayed in a variety of different formats, including but not limited to the autoregulation profile plot formats shown in FIGS. 6-8, and 15. In some embodiments, autoregulation data produced according to present disclosure embodiments may be displayed according to a mathematical model such as a sigmoidal function; e.g., the mathematical model may be fitted to the data for display purposes. A sigmoid function is a mathematical function having a characteristic "S"-shaped curve (sometime referred to as a "sigmoid curve"). An example of a sigmoid function that may be used with the present disclosure is as follows and is graphically depicted in FIG. 16:

$$S(x) = \frac{1}{(1+e^{\wedge}\{-x\})} \qquad [\text{Eqn. 3}]$$

As can be seen in FIG. 16, a sigmoidal curve has distinctive flat regions at two different values plus a curve region that is a transition zone between the two flat regions. In some embodiments of the present disclosure, a sigmoidal function can be used to mathematically fit autoregulation data. In the sigmoidal curve representation shown in FIG. 17, the AR Index increases as blood pressure drops below a lower autoregulation inflection point, as well as to separately fit autoregulation data wherein the AR Index increases as blood pressure increases above an upper autoregulation inflection point. In these embodiments, the lower and upper autoregulation inflection points may mark the range where the subject's autoregulation is functioning.

Non-linear regression techniques can be used to curve fit two different sigmoidal functions to autoregulation data at the lower and upper inflection points either in a single process or in a plurality of separate processes, with the separate results mathematically combined later. FIG. 17 illustrates an example of two different sigmoidal functions fit to autoregulation data (e.g., AR Index) as a function of mean blood pressure. In some embodiments, variables such as the upper flat region for the autoregulation profile plot may be constrained during the sigmoidal function fitting process to a value less than or equal to one (i.e., $\leq 1$) and the lower flat region for the autoregulation profile plot may be constrained to a value greater than or equal to zero (i.e., $\geq 0$). A non-limiting example of a linear regression process that may be used to fit sigmoidal functions is sequential quadratic programming (SQP), which is an iterative method for constrained nonlinear optimization.

In the example shown in FIG. 17, the model equation fit on the autoregulation data by non-linear regression consists of two sigmoidal functions:

$$AR[MBP] = \frac{(1-M)}{\left(1+e^{\wedge}\left(-\frac{(MBP-ZU)}{TU}\right)\right)} - \frac{(1-M)}{\left(1+e^{\wedge}\left(-\frac{(MBP-ZL)}{TL}\right)\right)} + 1 \qquad [\text{Eqn. 4}]$$

In this exemplary model equation, the term:

$$\frac{(1-M)}{\left(1+e^\wedge\left(-\frac{(MBP-ZU)}{TU}\right)\right)}$$

represents the upper mean blood pressure sigmoidal function, and the term:

$$-\frac{(1-M)}{\left(1+e^\wedge\left(-\frac{(MBP-ZL)}{TL}\right)\right)}+1$$

represents the lower MBP sigmoidal function. The parameter "MBP" represents the blood pressure bin, the parameter "M" represents the average or median of low AR Index values at MBP values between the lower and upper inflection points, which is usually representative of the flat part of the physiological autoregulation curve, the parameter "ZU" represents the upper sigmoidal function midpoint, the parameter "TU" represents the upper sigmoidal function curvature, the parameter "ZL" represents the lower sigmoidal function midpoint, and the parameter "TL" represents the lower sigmoidal function curvature. The parameters "M", "ZU", "ZL", "TU", and "TL" (fitting variables) may be solved by non-linear regression (NLR) and may be constrained to a limited range to help NLR converge to a solution. The "M" variable may be predetermined before NLR by pre-calculating the average or median of low AR Index values at MBP values between the lower and upper inflection points, which may further simplify NLR. Furthermore, the lower and upper sigmoidal functions in Eqn. 4 could be split at the midpoint of MBP (where AR Index values are lowest) and then processed independently with NLR. If the autoregulation profile plot shows the AR Index rising only at a low MBP, then the lower MBP sigmoidal function may be used in NLR to solve for the parameters "ZL" and "TL", and the upper MBP sigmoidal function may be dropped. Likewise, if the autoregulation profile plot shows the AR Index rising only at high MBP, then the upper MBP sigmoidal function may be used in NLR to solve for the parameters "ZU" and "TU", and the lower MBP sigmoidal function may be replaced by adding "M" to the equation.

When the autoregulation model of Eqn. 4 is solved by NLR, Eqn. 4 may be further manipulated into Eqn. 5 below with the same parameters to create an autoregulation curve that looks like the physiological textbook autoregulation curve shown in FIG. 3 by negating the lower sigmoidal function:

$$AR[MBP]=\frac{(1-M)}{\left(1+e^\wedge\left(-\frac{(MBP-ZU)}{TU}\right)\right)}+\frac{(1-M)}{\left(1+e^\wedge\left(-\frac{(MBP-ZL)}{TL}\right)\right)}+1-2(1-M) \qquad [Eqn.5]$$

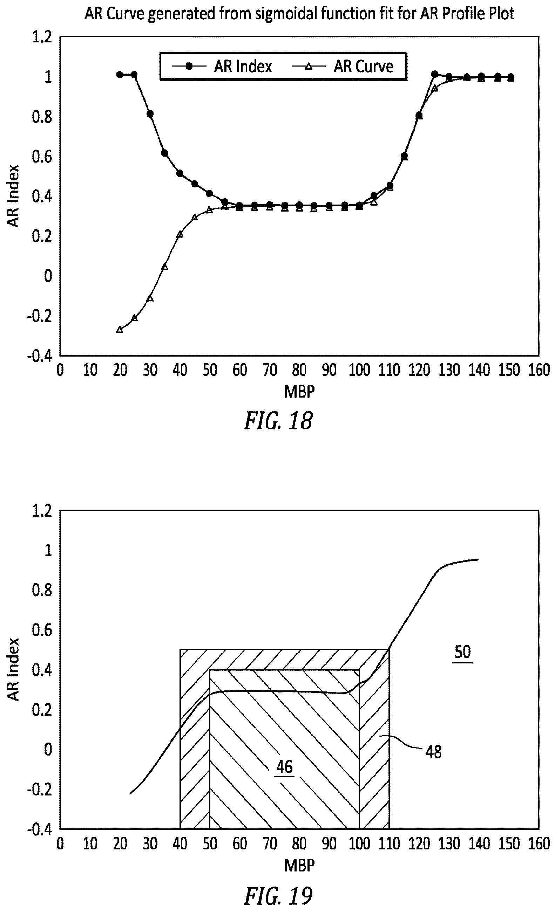

Replotting the autoregulation curve based on Eqn. 5 results in a physiological autoregulation representation (e.g., a curve) as shown in FIG. 18. The graphical representation shown in FIG. 18 (which the present AR system can be configured to display) can be displayed as an indicator for clinicians to understand the cerebral autoregulation state of the patient being monitored by real-time tissue oximeter and blood pressure monitoring. A graphical representation like that produced using Eqn. 5 (or similar equation) and shown in FIG. 18, may facilitate clinician understanding and provide ready interpretation due to its similarity to the curve configuration shown in FIG. 3 which is believed to be known in the art.

In the graphical representation shown in FIG. 18, the lower MBP inflection point that indicates the lower limit of autoregulation ("LLA") is between about 40-50 and the upper MBP inflection point that indicates the upper limit of autoregulation ("ULA") is between about 100-110.

The above description of mathematical modeling using sigmoidal functions (e.g., as indicated in Eqns. 3-5, and as shown in FIGS. 17 and 18) are non-limiting examples of how autoregulation data may be manipulated for display. The present disclosure is not limited to the specific equations described or the graphical representations shown; e.g., alternative sigmoidal functions and related displays are within the scope of the present disclosure.

In some embodiments of the present disclosure, the AM system controller 26 may be configured with instructions to examine (e.g., filter) autoregulation data prior to NLR curve fitting the data. For example, if the data (e.g., AR Index values) is unusually high for all MBP values, then the subject being monitored may have abnormal autoregulation function, or no autoregulation function. For example, if the lowest AR Indices calculated are higher than a predetermined threshold (e.g., AR Index values > 0.5), then the controller 26 instructions may instruct that no NLR curve fitting be performed, and in place of the fitted curve an indication of poor autoregulation function at all blood pressures may be provided (e.g., displayed).

In some embodiments of the present disclosure, the AM system controller 26 may be configured with instructions to produce a "simplified" indication of whether the current measured MBP is within the cerebral autoregulation limits. For example, the indication (e.g., displayed data) may include a physiological autoregulation curve (e.g., such as that shown in FIG. 18) and a graphic indication of whether the current measured MBP is within the cerebral autoregulation limits (e.g., determined from the autoregulation profile plot and/or derived physiological autoregulation curve). An example of such a graphic indication is shown in FIG. 19, which shows the current real-time MBP value in zones; a first zone 46 indicating that the MBP value is within the autoregulation zone (e.g., normal), a second zone 48 indicating that the MBP value is borderline (e.g., at the periphery of the autoregulation zone, but not yet outside the autoregulation zone), and a third zone 50 indicating that the MBP value is outside the autoregulation zone (e.g., abnormal). To facilitate quick recognition of these zones 46, 48, 50 by a clinician, the aforesaid zones may be color coordinated; e.g., the first zone 46 (autoregulation) may be colored an "acceptable" color (e.g., like green) to indicate normal conditions, the second zone 48 (borderline) may be colored a second color (e.g., a "caution" color like orange or yellow) to indicate borderline conditions, and the third zone 50 (borderline) may be colored a third color (e.g., a "warning" color like red) to indicate autoregulation passivity. In other words, the color scheme may provide a quickly recognizable information display to indicate the autoregulation function state wherein the subject's current MBP value resides.

As indicated above, mathematical modeling of autoregulation data and graphical representations thereof can provide useful information to a clinician. Autoregulation data modeling that utilizes sigmoidal functions is particularly useful. As disclosed above, inflection points within the sigmoidal curves can provide an indication of LLA and/or ULA position in terms of autoregulation index and blood pressure bin values. The inflection / LLA / ULA points may be used to define the subject's boundaries of pressure-independent blood flow (i.e., between the inflection points), and likewise the boundaries of pressure-passive blood flow (i.e., outside the inflection points). The specific location of an LLA or a ULA is not always clear from a visual inspection of the curve itself, however. Accurate LLA/ULA information (e.g., current AR status, AR trending, AR changes, etc.) representative of a recent period of time or over an extended period of time, or any combination thereof, can be invaluable to a clinician.

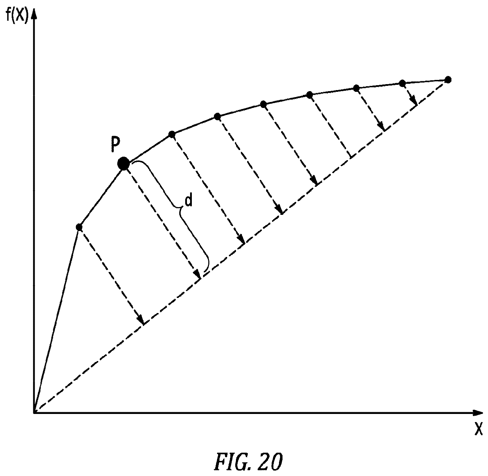

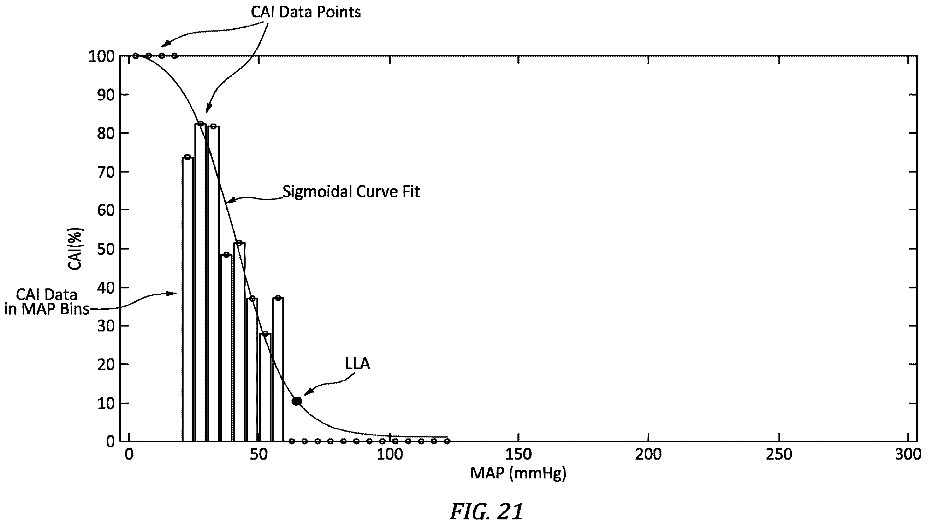

In some embodiments of the present disclosure, the AM system controller 26 may be configured to identify inflection / LLA / ULA points with a greater degree of certainty. For example, in instances where autoregulation data (e.g., cerebral autoregulation index ("CAI") or AR Index values based on COHZ, etc.) and corresponding blood pressure data (e.g., MBP bins) are determined and are mathematically modeled (as described herein), the inflection points of a fitted curve indicative of the subject's LLA and ULA may be identified based on the mathematical modeling itself. For example, slope values (i.e., first derivative) of a curve fitted to the AR data may be used to identify an inflection point. The identification may be based on the slope values themselves or based on a comparison of the slope values relative to a predetermined threshold value. Apart from slope, rate of change values (e.g., a second derivative of the curve) may be used to identify the inflection points associated with the LLA or ULA. The identification of the inflection points may be based on the rate of change values themselves (e.g., the maximum rate of change) or based on the rate of change values relative to a predetermined threshold value. Alternatively, an inflection point may be predicted as an "elbow point" on a fitted curve; e.g., for any the curve f(x), an "elbow point" methodology may be used to find a point "P" on the curve that has the maximum perpendicular distance "d" to a line joining the first and last points on the curve (e.g., see FIG. 20; Satopaa et al., "Finding a needle in a haystack: Detecting knee points in system behavior"; 2011 31st International Conference on Distributed Computing Systems Workshops, IEEE 2011). FIG. 21 illustrates a sigmoidal curve fit to CAI data as a function of binned blood pressure (MAP) values. The LLA as determined using an elbow point technique is shown on the curve in FIG. 21. Yet another technique for identifying inflection points (e.g., LLA) combines the use of slope and autoregulation data (e.g., CAI values, AR Index values, etc.) being within a predetermined threshold that is based on empirical data.

The predetermined thresholds may be based on empirical data that considers one or more of blood pressure, NIRS Index parameters, cerebral blood flow (CBF) data, vascular reactivity data, subject physiological parameters, and the like. The empirical data may be provided in a number of different formats. For example, empirical data may be statistically processed and organized in a manner that facilitates the determination of a predetermined threshold. The predetermined thresholds based on the empirical data may be in the form of a value or in a range of values. A specific non-limiting example of a predetermined threshold based on empirical data includes one or more NIRS Index values (as described above and shown in FIGS. 6-8) determined and organized as a function of blood pressure. The aforesaid NIRS Index / blood pressure data may be analyzed (e.g., via a curve-fitting process) to determine LLA and ULA points, and the predetermined thresholds may be based at least in part thereon. As another example, a predetermined threshold may be based on CBF data (e.g., CBF data determined using transcranial doppler techniques) empirically associated with LLAs and/or ULAs.

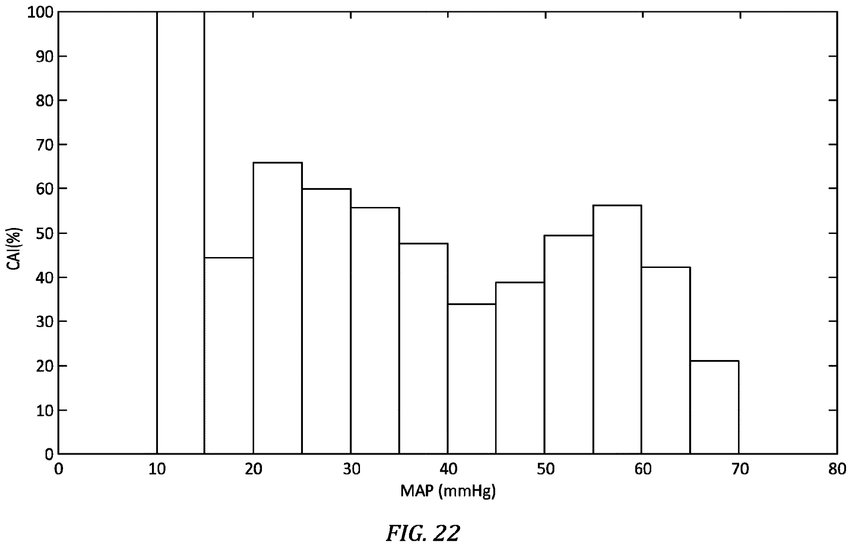

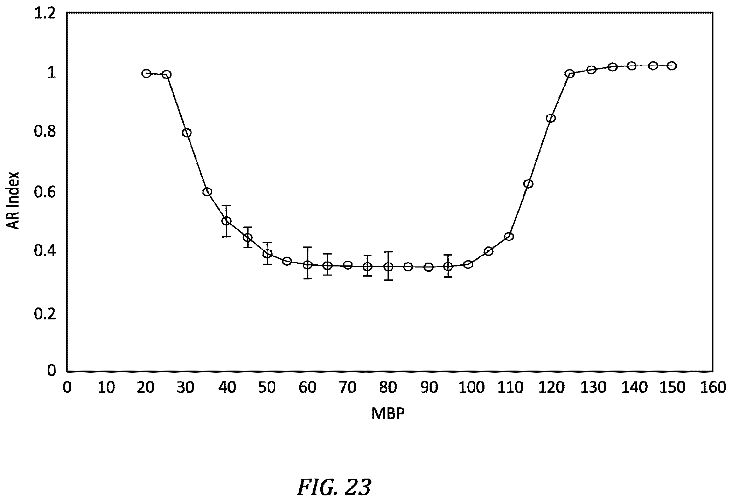

The description above and herein details that the autoregulation data (e.g., COHZ values, etc.), blood pressure data, and NIRS Index data (e.g., $StO_2$) used to determine a subject's LLA or ULA may filtered to remove discordant data and the filtered data may be processed to an average, a mean, a median, or similar collective value for each incremental blood pressure range (e.g., 0-20 mmHg, 20-25 mmHg, 25-30 mmHg, etc.). In some embodiments, the AM system controller 26 may be configured with instructions to determine a magnitude of variability present in the autoregulation data represented within each incremental blood pressure range. Referring to FIG. 22, a profile plot of CAI values versus binned blood pressure (MAP) is shown. The CAI values falling within each MAP bin may be recursively averaged over time. In addition to a mean or an average value, however, first order statistical information such as standard deviation or variance, a number of data points within a MAP bin, skewness, kurtosis, or percentile information may be determined. Such statistical values, individually or in any combination, may be used to determine the confidence of each average data point used for a curve fit. In some instances, the CAI data (or other AR data) in a particular bin may be skipped in the curve fitting process. For example, if a particular MAP bin has only a single CAI value binned, or a plurality of CAI values with a very high variance, then the CAI data within that MAP bin may be omitted in the curve fitting process. The magnitude of autoregulation data variability that may be present within an incremental blood pressure range can be useful as a confidence measure of the data. A low autoregulation data variability (e.g., a low standard deviation) may be expressed as the data having a higher level of confidence, and conversely a high autoregulation data variability (e.g., a high standard deviation) may be expressed as the data having a lower level of confidence. Alternatively stated, the data variability magnitude may be described as an indication of the uncertainty of the determined AR data (e.g., in the form of a fitted curve, etc.). The data variability magnitude may be reported / displayed in a variety of different ways, and the present disclosure is not limited to any particular method. In instances wherein an autoregulation curve is provided as a visual image, data variability may be displayed as part of that visual image. As an example, FIG. 23 illustrates vertical bars representative of the variability of the respective autoregulation data; e.g., the vertical length of the bar is indicative of the amount of variability. The visual indicators (i.e., the vertical bars) shown in FIG. 23 are non-limiting examples of how data variability may be visually indicated. In some embodiments, data variability may be reported numerically.

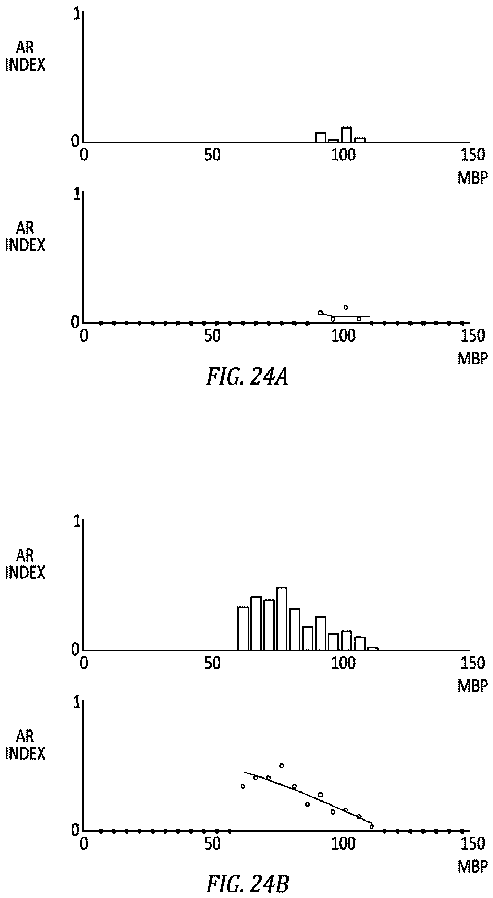

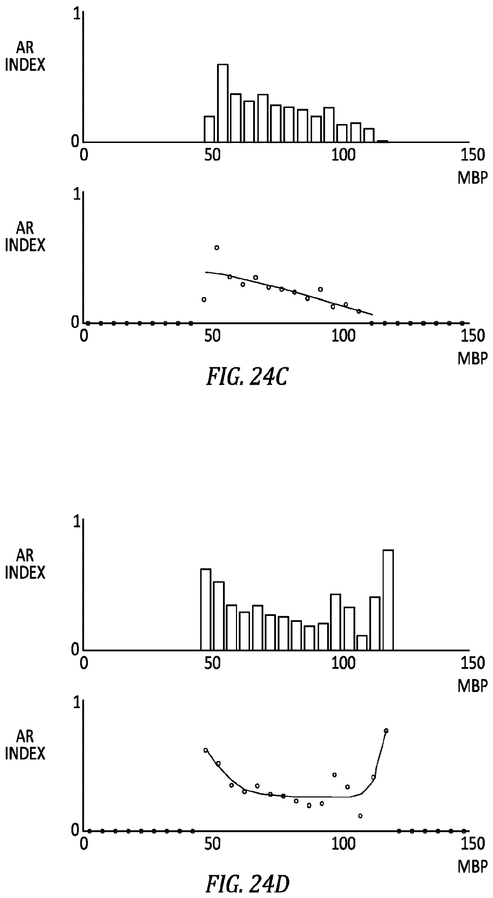

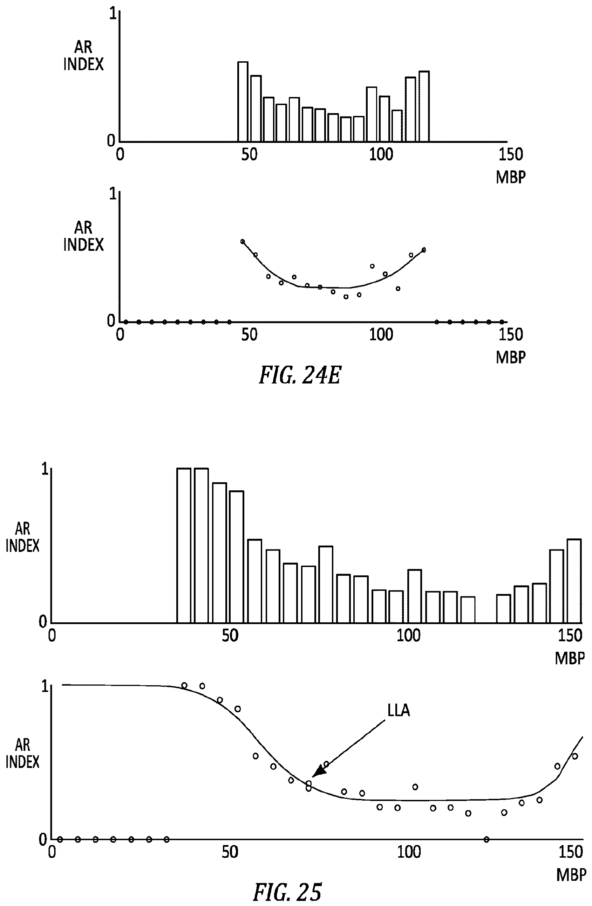

Some embodiments of the present disclosure AM system 20 may be configured (e.g., via stored instructions) to produce autoregulation data and curve fitting as a function of time; e.g., in real time. The graphs shown in FIGS. 24A-24E are representative still shots taken from a video illustrating an autoregulation curve (AR Index values along the Y-axis and blood pressure values (binned) along the X-axis) production as a function of time. In each of the graphs shown in FIGS. 24A-24E, there is an upper graph that shows a bar for each blood pressure bin with the height of the bar representative of the associated AR Index value for that blood pressure bin (e.g., an average AR Index value), and a lower graph that shows a data point for each AR Index / blood pressure bin value. In the lower graph, a curve is fitted to the plotted data points over time. Non-limiting examples of curve fitting techniques that may be used are described herein. The graphs illustrate the data points and curve fitting sequentially from the start of monitoring: FIG. 24A illustrates the data plotted shortly after the start; FIG. 24B illustrates data collected from start to about three minutes; FIG. 24C illustrates data from start to about seven minutes; FIG. 24D illustrates data collected from start to about nine minutes; and FIG. 24E illustrates data collected from start to about ten and one-half minutes. As indicated above, autoregulation data (e.g., AR Index, CAI, COHZ values, etc.) may be processed as an average, a mean, a median, or similar collective value for each incremental blood pressure range (e.g., 0-20 mmHg, 20-25 mmHg, 25-30 mmHg, etc.). The autoregulation data shown in FIGS. 24A-24E is processed in this manner to illustrate a real-time production of autoregulation data. As can be seen in FIG. 24A, a limited amount of AR Index data is available in a few blood pressure bins based on the very short period of monitoring. FIGS. 24B-24E represent substantially more autoregulation data associated with a far greater number of blood pressure bins. As can be seen in the example shown in FIGS. 24A-24E, the AR Index values in the respective bins vary over time (e.g., from T=0 to T=10:30 minutes). The changes in AR Index are a function of the average value of the AR Index data over the respective period of time from the start of monitoring.

In some embodiments, the AM system 20 may be configured (e.g., via stored instructions) to produce and display autoregulation data produced in several different modes. In a first mode, the system 20 may be configured to produce and display autoregulation data representative of the entirety of the monitoring period. In a second mode, the AM system 20 may be configured to produce and display autoregulation data collected over a selected period of time; e.g., a "truncated" period of time less than the entire monitoring period such as data collected over the last hour, or the last five hours, etc. In some embodiments, the truncated period of time may be very brief; e.g., a "start-up" period of one or more minutes that avoids any substantial initial data buffering. A display showing such a brief start-up period can provide information quickly and avoid any lapse in autoregulation data reporting from the onset of monitoring that may occur if the data is buffered. In a third mode, the AM system 20 may be configured to produce and display data collected over the entirety of the monitoring period and data collected over a truncated period of time or be configured to permit the user to selectively switch between those modes. In some embodiments, the AM system 20 may be configured to produce and display data in a default normal mode representative of data collected over the entirety of the monitoring period. In these embodiments, the AM system 20 may be further configured to display autoregulation representative of a preselected truncated period of time upon the occurrence of an event. For example, if the COHZ values / AR Index values produced in a recent period of time (e.g., the last ten minutes, etc.) abruptly change in a concerning manner (e.g., reach or exceed a predetermined threshold value, etc.), the AM system 20 may be configured to produce and display autoregulation data collected over the preselected truncated period of time (e.g., the last hour, etc.). The aforesaid autoregulation data collected over the preselected truncated period of time may replace the "normally" shown autoregulation data, or may be displayed coincident with the normally shown autoregulation data, or the system may be configured to produce a "flag" that alerts the user to switch the display mode from the normal mode to display data collected over the truncated period of time. In this manner, the recent autoregulation data that may give rise to the concern would not be subject to long term averaging, and therefore would not be subject to long term averaging / dilution.

In those embodiments of the present disclosure AM system 20 that are configured to produce autoregulation data and curve fitting as a function of time (e.g., in real time such as the example shown in. FIGS. 24A-24E), the LLA and/or ULA points may also be determined and shown when sufficient autoregulation data has been collected and curve fitting has occurred. Example methodologies of determining the LLA and/or ULA points are described above. In similar fashion to the modes of producing and displaying autoregulation data described above, the LLA and/or ULA points may be determined and displayed (see FIG. 25); e.g., based on autoregulation data representative of the entirety of the monitoring period, and/or based on autoregulation data collected over a truncated period of time such as the last hour etc. The ability to display LLA and/or ULA points in real-time based on autoregulation data representative of the entirety of the monitoring period, and/or based on autoregulation data collected over a truncated period of time is understood to be useful; e.g., to facilitate the identification of LLA changes, etc.

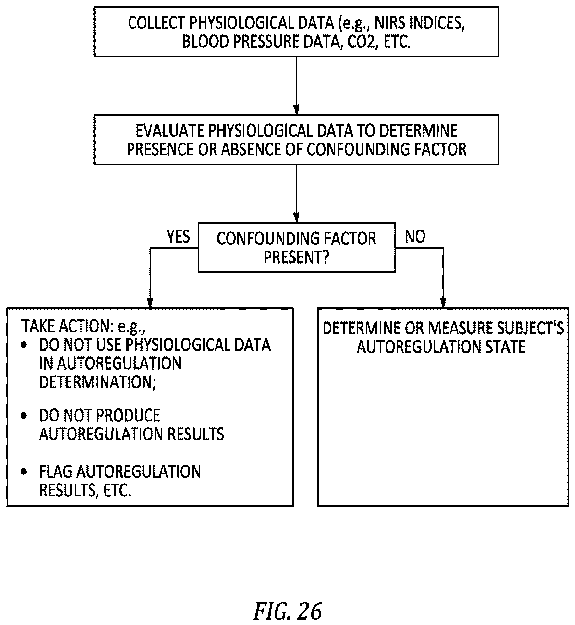

Referring to FIG. 26, in some embodiments of the present disclosure the AM system controller 26 may be configured to identify the presence or absence of a "confounding factor" that may detrimentally affect the validity of a determination (which determination may include a measurement of the same) of a subject's autoregulation state. The term "confounding factor" as used herein refers to a physiological condition that affects one or more physiological parameters (e.g., NIRS index, blood pressure, heart rate of the subject, or the like, or any combination thereof) in a manner independent of the subject's autoregulation state. If such a physiological condition is left unaccounted for, the validity of the determination of the subject's autoregulation state can be detrimentally affected. The confounding factors include, but are not limited to, hypoxia, pain, $CO_2$ level (hypercapnia and hypocapnia), physiological uncertainty, scalp/extracerebral blood flow changes, and venous congestion. If physiological data indicative of a confounding factor is identified, the AM system controller 26 may be configured to act; e.g., disregard that data, flag the data as suspect, not produce the determination of a subject's autoregulation state using that data, or the like.

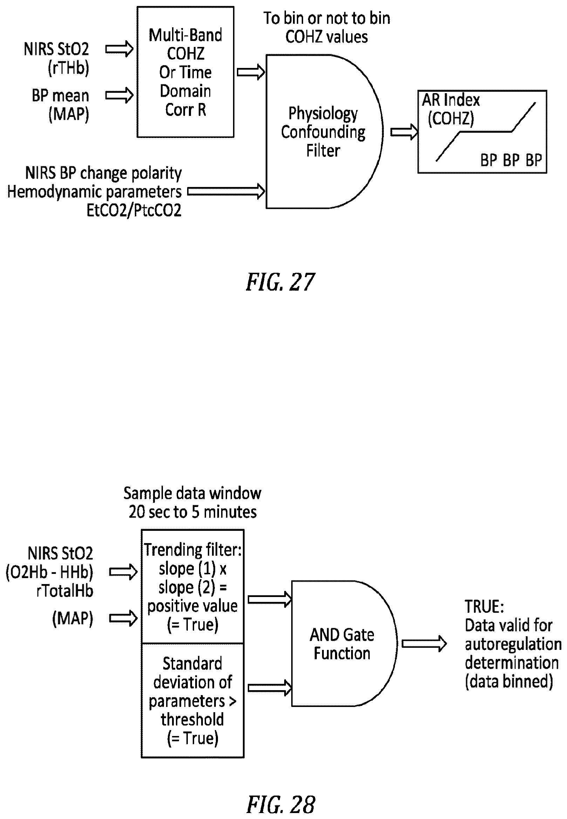

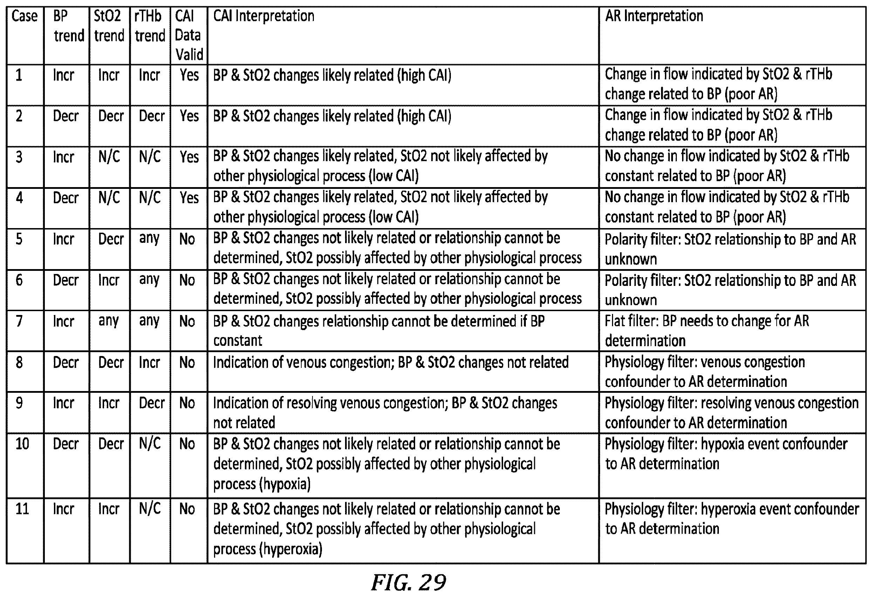

FIG. 27 illustrates an exemplary logic diagram of how the presence or absence of a confounding factor may be determined under the present disclosure. In this example, NIRS index data (e.g., $StO_2$, rTHb, etc.) and mean arterial blood pressure (MAP) data collected over a predetermined period are each respectively organized. The aforesaid data may be organized within a time domain and processed using a correlation technique, or organized within a time domain, subsequently transformed into a frequency domain, and the frequency domain data used to produce COHZ values determined in a manner similar to that described above; e.g., see FIGS. 5 and 9 and the descriptions associated therewith. The time domain - correlated data or the frequency domain - COHZ data may then be input into a physiology confounding filter. Contemporaneously, other data (e.g., NIRS parameter or blood pressure trend values, or hemodynamic parameters, or carbon dioxide data, such as end tidal carbon dioxide values - $EtCO_2$, and/or transcutaneous carbon dioxide values - $PtcCO_2$, etc.) may also be input into the physiology confounding filter. The aforesaid physiology confounding filter may be configured to evaluate the significance of the input data. If the filter determines that the input data is independent of (which may also be described as being unrelated to) the subject's autoregulation function (e.g., the magnitude of the parameter is inconsistent with the subject's autoregulation state, or a plurality of parameters provide conflictual information relating to the subject's autoregulation state such as opposite trend data, etc.) then the presence of a confounding factor may be determined. As a result of the presence of a confounding factor being determined, the input data may not be used in a determination of the subject's autoregulation state, or any autoregulation data produced using the data may be flagged as potentially suspect, or autoregulation data may not be produced, or the like. The table shown in FIG. 29 is an example of logic table that may be the basis for a physiology confounding filter for evaluating trends between $StO_2$ and rTHb and the significance of those trends with respect to an autoregulation analysis.

NIRS indices such as $StO_2$ and/or MAP can change, or they may trend in opposite directions, and that change(s) or opposing trend may be a result of a factor that is independent of a subject's autoregulation state. In other words, a confounding factor can cause a subject's $StO_2$, and/or MAP to change, or oppositely trend, and that change or opposite trend is not a result of the subject's autoregulation function. Some prior art publications suggest that autoregulation state determinations are unaffected by $StO_2$ and MAP values changing / trending in opposite directions. However, there is uncertainty regarding the validity of this assumption, and more particularly uncertainty regarding whether a $StO_2$ value trend opposite a MAP value trend is confounded by a different physiological process. Hence, the assumption that autoregulation state determinations are unaffected by $StO_2$ and MAP values changing / trending in opposite directions could lead to a false positive of an acceptable autoregulation state, when in fact the $StO_2$ changes may be independent of and not related to the subject's autoregulation state.

FIG. 28 illustrates an exemplary functional diagram of how the NIRS index (e.g., $StO_2$) data and MAP data may be used to identify the presence or absence of a physiological uncertainty as a confounding factor, and whether the NIRS index data should be used in the autoregulation state determination. For example, the present disclosure may be configured to determine the presence of a physiological uncertainty (e.g., a confounding factor) by sensing the subject's $StO_2$ and MAP over a predetermined period of time and create "trend data" for the sensed $StO_2$ and MAP values. The duration of the predetermined period can be varied to suit the application, but a predetermined period of time as little as twenty seconds to as much as five minutes is believed to be acceptable. The term "trend data" is used here to refer to

changes in the StO$_2$ values and MAP values over the predetermined period of time. For example, the StO$_2$ values and MAP values collected periodically over the period of time may be mathematically evaluated using a regression line fit analysis to produce a regression line slope for the respective NIRS index. The regression line slope of the StO$_2$ values and the MAP values versus the predetermined period of time may then be comparatively analyzed. For example, the slope values may be multiplied, and if the product of the slope values multiplication is positive, then the aforesaid product would indicate that the StO$_2$ values and the MAP values being compared are trending in the same manner. The multiplication of the regression lines slopes may be considered to be a type of "polarity filter". StO$_2$ values and MAP values trending in the same manner (e.g., both along a positive or a negative slope) indicate that the StO$_2$ values and the MAP values are generally in agreement with one another (e.g., a normal condition), whereas StO$_2$ values and MAP values trending in opposite directions (e.g., one having a positive slope, the other having a negative slope) may indicate a physiological uncertainty. Analytical tools other than a regression line fit analysis may be used to evaluate the StO2 values and the MAP values. The present disclosure is not limited to using StO$_2$ values and MAP values for evaluating trends, and/or not limited to using StO$_2$ values and MAP values as the basis for a polarity filter.

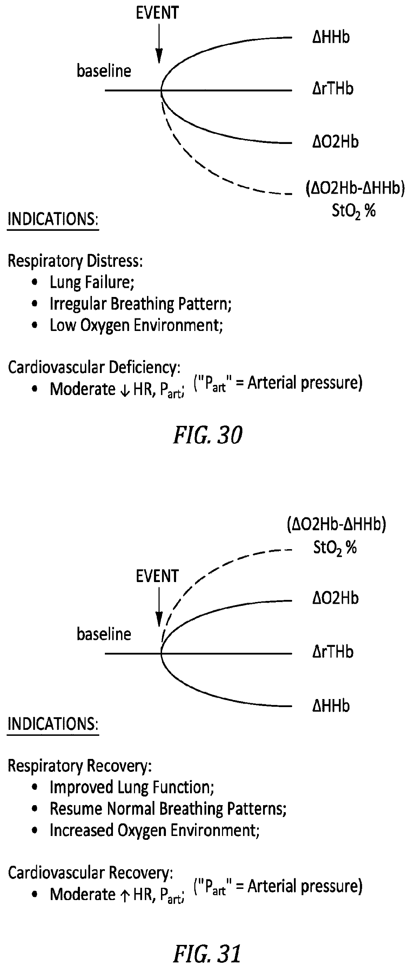

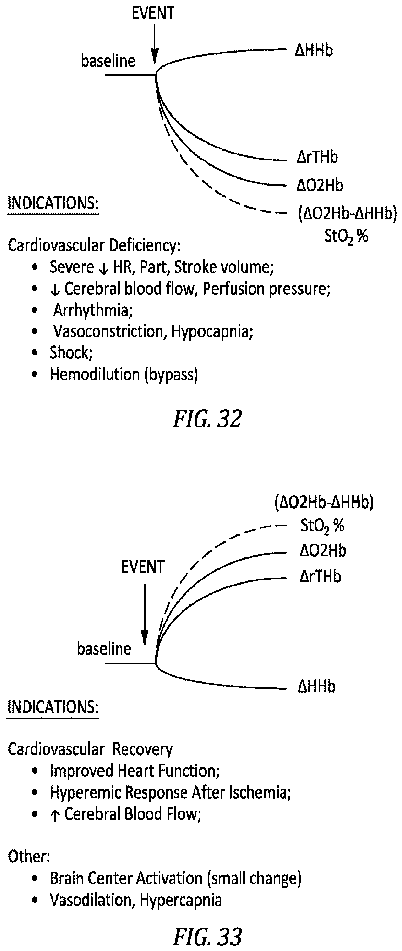

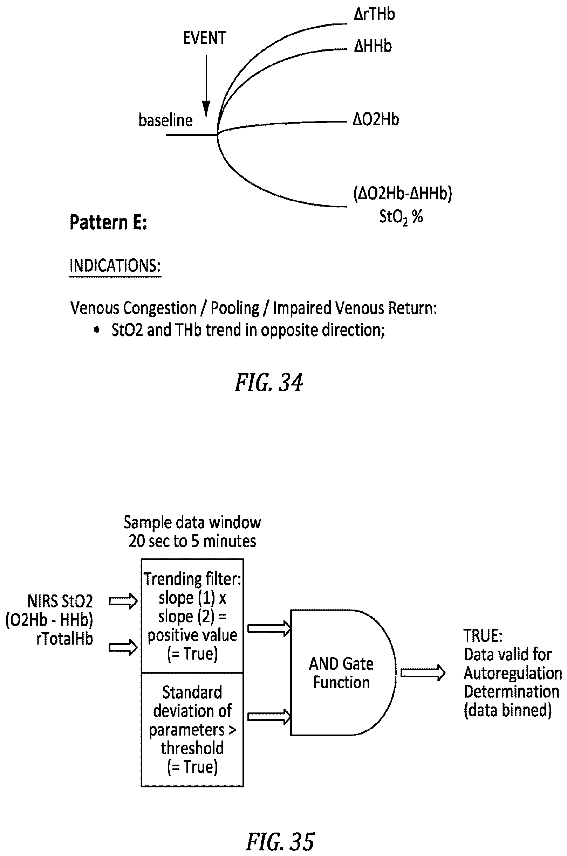

FIG. 29 is an example of a multiple trend input table that may be used as a basis for a polarity filter for determining whether NIRS index values (and COHZ values based thereon) are useful for determining autoregulation information based on their respective trends ("Inrc" = increasing trend; "Decr" = decreasing trend; "N/C" = no change; and "any" = any of an increasing or decreasing trend, or no change in trend) relative to the associated trend in blood pressure. The example table shown in FIG. 29 includes a substantial number of BP, StO$_2$, and THb trend variations for illustration purposes. Polarity filters may include fewer or more BP, StO$_2$, and rTHb trend variations and may include other NIRS index values in addition to or in place of StO$_2$ and rTHb. As can be seen from FIG. 29, the usefulness/validity of NIRS index data (e.g., whether the NIRS index data is independent of autoregulation or related to autoregulation) can be readily evaluated (e.g., "CAI Data Valid") when the same is considered relative to the associated trend in blood pressure. If the relative trends are such that the NIRS index data is attributable to autoregulation, then the NIRS index data is valid for use in an autoregulation data determination. For example, in Case 1 BP, StO$_2$, and rTHb respective collected data are all trending in an increasing manner. In this instance, the BP and StO$_2$ changes are likely related and the change in blood flow indicated by the increased trends in StO2 and THB and the increase trend in BP, are likely indicative of poor autoregulation. Conversely, in Case 8 BP and StO$_2$ are on decreasing trends but the rTHb is on an increasing trend. In this instance, the relative trends of BP and StO$_2$ are not likely related and may be an indicator of venous congestion. Hence, the NIRS index data is not likely attributable to autoregulation and the NIRS index data is not valid for use in an autoregulation data determination. In Case 7, the BP is on a flat trend (i.e., N/C). Since any autoregulation determination requires a changes in blood pressure, the NIRS index data trends in this case are not likely attributable to autoregulation and the NIRS index data is not valid for use in an autoregulation data determination. As can be seen from the above, the relative trends of one or more NIRS indices and BP can provide a means for determining the presence or absence of a physiological uncertainty (e.g., a confounding factor) and filter. One of numerous advantages provided by a trend input table / filter / confounding factor determination as described above is its ability to distinguish NIRS index data attributable to certain clinical circumstances from NIRS index data attributable to autoregulation. As stated above, the presence of venous congestion may provide NIRS trend data that can be misinterpreted as being associated with autoregulation. Venous congestion may occur, for example, when a catheter impedes or blocks venous blood flow, resulting in a backing up or pooling of blood within a region of a subject's body, particularly the brain. The present disclosure provides an improved method for distinguishing such data. FIGS. 30-34 provide a visual representation of possible NIRS Index trends in response to a physiological event. FIGS. 30 and 31 illustrate trend reactions to a physiological event such as respiratory changes where the StO$_2$ trend changes but the rTHb trend remains substantially constant / no change. The NIRS Index trends shown in FIG. 30 may be associated with respiratory distress and cardiovascular deficiency. The NIRS Index trends shown in FIG. 31 may be associated with respiratory and cardiovascular recovery. FIGS. 32 and 33 illustrate trend reactions to a physiological event such as blood flow changes and cardiovascular changes (e.g., ischemia, vasoreactivity, blood hemoglobin - HGB, etc.) where StO2 and rTHb trend in the same direction. The NIRS index trends shown in FIG. 32 may be associated with cardiovascular deficiency and the NIRS index trends shown in FIG. 33 may be associated with cardiovascular recovery. FIG. 34 illustrates trend reactions (e.g., StO2 and rTHb) to a physiological event such as obstructed venous blood circulation, where StO2 and rTHb trend in opposite directions.

This example of determining the presence or absence of a physiological uncertainty as a confounding factor may also evaluate the magnitude of change of the factor being considered; i.e., if the confounding factor being considered evidences a change, but that change is minimal (e.g., below a threshold amount), then the confounding factor is likely to be not clinically relevant. An example of a "magnitude of change" evaluation may utilize a standard deviation (or other statistical parameter) determination of the StO$_2$ and MAP values being compared. If the standard deviation of both the StO$_2$ and MAP values being compared are above respective predetermined thresholds, then the aforesaid standard deviation values would indicate that the magnitude of the StO$_2$ and MAP values are clinically meaningful. Standard deviation values below the predetermined threshold would indicate that the trending is not clinically meaningful. The aforesaid standard deviation determination, which may be considered to be a type of "change of magnitude filter", is an example of a "magnitude of change" evaluative technique and the present disclosure is not limited thereto.

The StO$_2$ and MAP trend data and magnitude change data may then be input into a logic gate (e.g., an AND gate) to determine the presence or absence of a physiological uncertainty as a confounding factor, and therefore whether the collected StO$_2$ data and the MAP data are acceptable or not acceptable for a determination of the subject's autoregulation state. The logic diagram shown in FIG. 28 represents an exemplary mechanism for determining the presence or absence of a physiological uncertainty as a confounding factor in terms of StO$_2$ and MAP and the present disclosure is not limited thereto. To be clear, the logic diagram shown in FIG. 28 is a simplistic representation provided for illustrative purposes. As stated above, a logic gate / filter as diagrammatically shown may include a variety of trend variations (e.g., see FIG. 29) that may utilize NIRS indices in addition to or other than StO$_2$ and rTHb.

If a subject is experiencing hypoxia, or is recovering from hypoxia, or is given supplemental oxygen, or the like, one or more NIRS indices sensed by the tissue oximeter 24 will likely be substantially independent of the subject's autoregulation state. As a result, any autoregulation state determination using the aforesaid independent NIRS index(ices) will likely detrimentally affect the validity of an autoregulation state determination. To avoid such a detrimental effect, embodiments of the present disclosure may include sensing the subject to determine whether the subject is hypoxic, or trending or recovering from hypoxia, or has been given supplemental oxygen, or the like. If the subject is hypoxic, or is recovering from hypoxia, or has been given supplemental oxygen, or the like, embodiments of the present disclosure may act in view thereof (e.g., via algorithmic instructions), which actions may include discarding (e.g., not binning) data produced while the subject is hypoxic, flagging or not producing autoregulation state determination data, or the like. The present disclosure is not limited to any particular technique for determining hypoxia. As an example, embodiments of the present disclosure may utilize the tissue oximeter 24 to sense one or more NIRS indices and compare the determined NIRS index value(s) to an appropriate corresponding threshold value to determine if the subject is hypoxic or not; e.g., using stored algorithmic instructions.

FIG. 35 illustrates an exemplary functional diagram of how NIRS index trend data may be used to identify the presence or absence of hypoxia as a confounding factor, and subsequently whether the NIRS index data should be used in the determination of autoregulation state. For example in a manner similar to that described above, the present disclosure may be configured to determine the presence of hypoxia, or to determine if the subject is recovering from hypoxia, or whether the subject has been given supplemental oxygen, or the like, by sensing at least two NIRS indices (e.g., StO$_2$, rTHb) over a predetermined period of time and create trend data for each respective NIRS index; e.g., the respective NIRS index values collected periodically over a period of time may be mathematically evaluated using a regression line fit analysis and the determined slopes of the respective NIRS index regression lines versus the predetermined period of time may then be comparatively analyzed. During the onset of hypoxia or when a subject is recovering from hypoxia, or has been given supplemental oxygen, the subject's StO$_2$ tends to change but the rTHb remains relatively stable (zero slope vs. time). NIRS indices trending in the same manner (e.g., both along a positive or a negative slope) indicate that the NIRS indices are in agreement, likely relate to the subject's autoregulation system, and therefore the collected NIRS data would be acceptable for an autoregulation state determination. NIRS indices trending in opposite directions (e.g., one positive slope, the other negative slope), on the other hand, indicate that at least one of the NIRS indices is independent of the subject's autoregulation system, and therefore the collected NIRS data would be unacceptable for an autoregulation state determination. Also similar to the processes described above, embodiments of the present disclosure evaluating hypoxia as a possible confounding factor may also evaluate the magnitude of change of the factor being considered; i.e., if the relevant parameters being considered evidence a change, but that change is minimal (e.g., below a threshold amount), then the confounding factor is likely to be not clinically relevant. The product of the regression line slopes and the standard deviation values may be input to a logic gate (e.g., an "AND gate") or other trending filter. If the product of the regression line slopes indicates the compared NIRS indices (e.g., StO$_2$, rTHb) are trending in the same manner (i.e., a positive value), and the standard deviation values indicate trends of sufficient magnitude, then both represent a "true" input to the logic gate and the NIRS index data is determined to be useful in a determination of the subject's autoregulation state; i.e., the absence of a hypoxia as a confounding factor. Conversely, if the product of the regression line slopes does not indicate that the NIRS indices are trending in the same manner (i.e., a negative value, or a near zero value), and/or the standard deviation values do not indicate trends of sufficient magnitude, then either or both represent a "false" input to the logic gate, and presence of hypoxia as a possible confounding factor is determined, and the NIRS index data may not be used or is flagged in the subject's autoregulation state determination.

Damaging or potentially damaging stimuli experienced by a subject may cause nociceptors to produce signals, which signals in turn produce one or more physiological responses (e.g., pain) to address the damaging or potentially damaging stimuli. At least some of these physiological responses can involve the same parameters that may be used to evaluate autoregulation; e.g., heart rate, blood pressure, NIRS indices, etc. Hence, a subject experiencing pain may exhibit changes to certain physiological parameters and these changes may be independent of the subject's autoregulation state. If not accounted for, these physiological parameters may negatively affect the accuracy of an autoregulation state determination of the subject.

Some embodiments of the present disclosure may be configured to determine whether the subject is experiencing pain and based on that determination subsequently determine whether the collected physiological data (e.g., blood pressure data, NIRS index data, etc.) should be used in an autoregulation state determination.

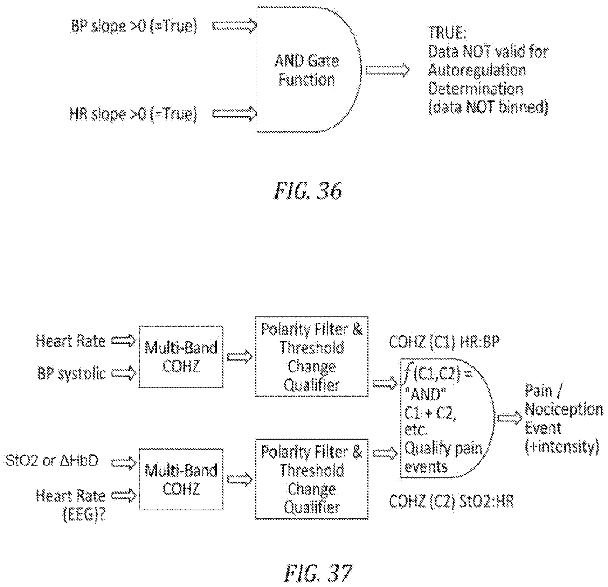

Non-limiting examples of determining the presence or absence of pain as a confounding factor are diagrammatically shown in FIGS. 36-38. In the example diagrammatically shown in FIG. 36, heart rate data and systolic blood pressure data may be collected over a predetermined period of time. The aforesaid heart rate and systolic blood pressure data may be collected over exactly the same predetermined period of time, or substantially the same period of time. The aforesaid heart rate and systolic blood pressure data may be mathematically evaluated to determine whether the respective parameter is increasing over the predetermined period of time; e.g., the respective data may be analyzed using a regression line fit analysis to produce a regression line slope. If the heart rate data and systolic blood pressure data are both positively trending (increasing), that would be an indication that the subject may be experiencing pain and therefore the presence of pain as a confounding factor. As described herein, the trend data for each parameter may be input to a logic gate (e.g., an "AND gate" as shown in FIG. 36) configured to determine the presence or absence of the confounding factor, and therefore whether the collected data is acceptable for an autoregulation state determination. The logic diagram shown in FIG. 36 represents an exemplary mechanism for determining the presence or absence of pain as a confounding factor in terms of the heart rate and systolic blood pressure data and the present disclosure is not limited thereto.

In the example diagrammatically shown in FIG. 37, heart rate data, systolic blood pressure data, and NIRS index data may be collected over a predetermined period of time. The aforesaid heart rate, systolic blood pressure, and NIRS index data may be collected over exactly the same predetermined period of time, or substantially the same period of time. The present disclosure is not limited to any particular technique for determining the subject's heart rate; e.g., an electrocardiogram (ECG) may be used.

In the non-limiting example diagrammatically shown in FIG. 37, heart rate data and systolic blood pressure data collected over the predetermined period may each respectively be organized within a time domain, subsequently transformed into a frequency domain, and the frequency domain data used to produce COHZ values determined in a manner similar to that described above; e.g., see FIGS. 5 and 9 and the descriptions associated therewith. The COHZ values may then be evaluated using a polarity filter and a change magnitude filter to determine if the subject's systolic blood pressure and heart rate are indicative of a pain response. For example, if the COHZ values indicate that the subject's heart rate and systolic blood pressure are both trending in the same manner (i.e., both positive values), and standard deviation analyses of the values indicate trends of sufficient magnitude, then both may indicate the presence of pain as a confounding factor. Conversely, if the COHZ values indicate that the subject's heart rate and systolic blood pressure are not both trending in the same manner, and/or standard deviation analyses of the subject's heart rate and systolic blood pressure values do not indicate trends of sufficient magnitude, then these parameters may not indicate pain as a confounding factor. In similar fashion, the subject's NIRS index data (e.g., $StO_2$, HbD, etc.) and heart rate data collected over the predetermined period may be each respectively organized within a time domain, transformed into a frequency domain, and the frequency domain data used to produce COHZ values determined in a manner similar to that described above. The COHZ values may then be evaluated using a polarity filter and a change magnitude filter to determine if the subject's NIRS index and heart rate indicate the presence of pain as a confounding factor. For example, if the COHZ values indicate that the subject's NIRS index and heart rate are both trending in the same manner (i.e., positive values), and standard deviation analyses of the values indicate a trend of sufficient magnitude, then both may indicate the presence of pain as a confounding factor. Conversely, if the COHZ values indicate that the subject's NIRS index and heart rate are not both trending in the same manner, and/or standard deviation analyses of the subject's NIRS index and heart rate values do not indicate a trend of sufficient magnitude, then these parameters may not indicate the presence of pain as a confounding factor. The heart rate and systolic blood pressure COHZ trend data and magnitude change data, and the NIRS index and heart rate COHZ trend data and magnitude change data may then be input into a logic gate (e.g., an AND gate) to determine the presence or absence of pain as a confounding factor, and therefore whether the collected NIRS index data and systolic blood pressure data is acceptable for a determination of the subject's autoregulation state. The logic diagram shown in FIG. 37 represents an exemplary mechanism for evaluating pain in terms of the heart rate, systolic blood pressure data, and NIRS parameters and the present disclosure is not limited thereto. In addition, the above example utilizes a frequency domain and COHZ values approach. As indicated herein, the present disclosure is not limited to utilizing a frequency domain and COHZ values approach; e.g., a time domain and correlation technique may be used.

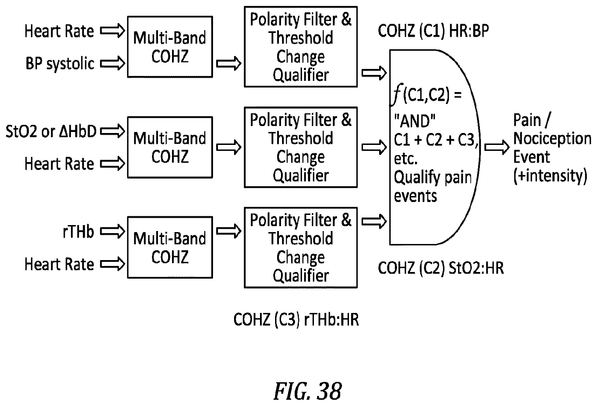

In the example diagrammatically shown in FIG. 38, an analysis methodology similar to that described above in terms of FIG. 37 is used, and in this instance an additional evaluative parameter is added. Specifically, in this example the additional evaluative parameter utilizes an additional NIRS index data (e.g., rTHb) and heart rate data collected over the predetermined period. Here again, both parameters may each respectively be organized within a time domain, transformed into a frequency domain, and the frequency domain data used to produce COHZ values determined in a manner similar to that described above. The COHZ values may then be evaluated using a polarity filter and a change magnitude filter to determine if the subject's NIRS index (e.g., rTHb) and heart rate are indicative of a pain response. For example, if the COHZ values indicate that the subject's NIRS index (e.g., rTHb) and heart rate are both trending in the same manner (e.g., positive values), and a standard deviation analysis of the values indicate a trend of sufficient magnitude, then both are indicative of pain as a confounding factor. Conversely, if the COHZ values indicate that the subject's NIRS index (e.g., rTHb) and heart rate are not both trending in the same manner, and/or a standard deviation analysis of the subject's NIRS index and heart rate values do not indicate a trend of sufficient magnitude, then these parameters are not indicative of pain as a confounding factor. The: a) heart rate and systolic blood pressure COHZ trend data and magnitude change data; b) NIRS index ($StO_2$, HbD) and heart rate COHZ trend data and magnitude change data; and c) NIRS index (rTHb) and heart rate COHZ trend data and magnitude change data, may then be input into a logic gate (e.g., an AND gate) to determine if the collected heart rate, NIRS index data, and systolic blood pressure data indicate pain as a confounding factor, and therefore whether the data is acceptable for a determination of the subject's autoregulation state. The logic diagram shown in FIG. 38 represents an exemplary mechanism for evaluating pain as a confounding factor in terms of the heart rate, systolic blood pressure data, and NIRS data, and the present disclosure is not limited thereto.

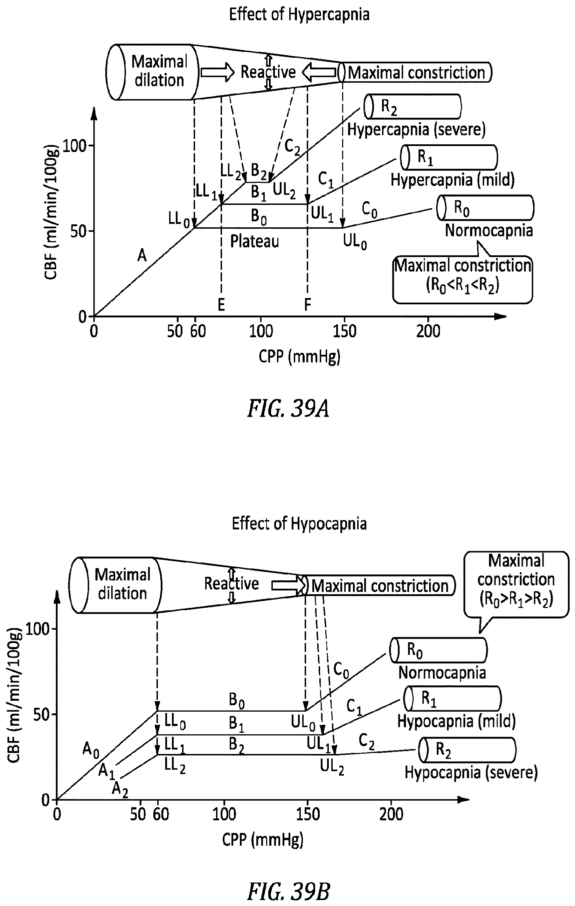

Carbon dioxide ($CO_2$) within blood affects cerebrovascular reactivity independently of cerebral perfusion pressure. The shape of a cerebral autoregulation curve, including the lower limit of autoregulation (LLA) and the upper limit of autoregulation (ULA), can change if the $CO_2$ level within the blood is outside of its normal range (normocapnia); e.g., if the subject is experiencing hypercapnia or hypocapnia. FIG. 39A is a graph of cerebral blood flow (CBF) versus cerebral perfusion pressure (CPP) illustrating the effects of hypercapnia. FIG. 39B is a graph of cerebral blood flow (CBF) versus cerebral perfusion pressure (CPP) illustrating the effects of hypocapnia. (Graphs from L. Meng and A.W. Gelb, *"Regulation of Cerebral Autoregulation by Carbon Dioxide"*, Anesthesiology, 2015) The hypercapnia graph in FIG. 39A shows a significant difference between the LLA and ULA in a mild hypercapnia state versus the LLA and ULA in a normocapnia state, and an even more significant difference between the LLA and ULA in a severe hypercapnia state versus the LLA and ULA in a normocapnia state. The hypocapnia graph in FIG. 39B shows a significant difference between the ULA in a mild hypocapnia state versus the ULA in a normocapnia state, and an even more significant difference in the ULA in a severe hypocapnia state versus the ULA in a normocapnia state. Hence, if a subject's blood $CO_2$ level is outside of its normal range and that condition is not accounted for, the validity of the subject's autoregulation state determination can be detrimentally affected.

Some embodiments of the present disclosure may be configured to determine if a subject's blood $CO_2$ level is abnormal and therefore may be a confounding factor, and based on that determination determine whether the collected physiological data (e.g., blood pressure data, NIRS index data, etc.) should be used in the determination or measurement of autoregulation.

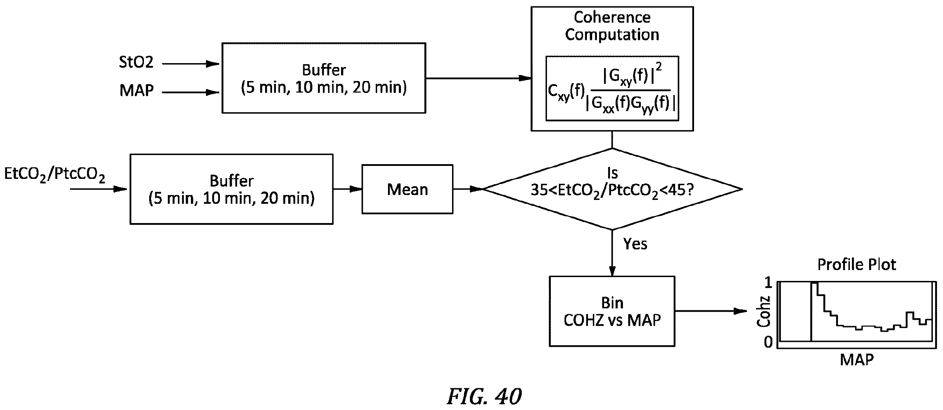

In a first example diagrammatically shown in FIG. 40, NIRS index data (e.g., $StO_2$), MAP data, and $CO_2$ level data (e.g., end-tidal $CO_2$ - "$EtCO_2$", or, - transcutaneous $CO_2$ - "$PtcCO_2$", or the like) may be collected over a predetermined period of time. The aforesaid NIRS index data, MAP data, and blood $CO_2$ data may be collected over exactly the same predetermined period of time, or substantially the same period of time. The present disclosure is not limited to any particular technique for determining the subject's blood $CO_2$ data. The collected $StO_2$ and MAP data may be organized within a time domain and processed using a correlation technique, or organized within a time domain, subsequently transformed into a frequency domain, and the frequency domain data used to produce COHZ values determined in a manner similar to that described above; e.g., see FIGS. 5 and 9 and the descriptions associated therewith. The $CO_2$ level data collected during the predetermined period of time may be mathematically evaluated to determine a mean value. The term "mean" as used here contemplates that a number is produced that is representative of the sensed $CO_2$ values determined during the predetermined period of time. The term "mean" is not limited to an arithmetic mean (i.e., the sum of the numbers divided by how many numbers are being averaged), and alternatively may be another measure of central tendency, such as an average, a median, a mode value, or the like. The $CO_2$ level data may then be evaluated to determine if the subject's blood $CO_2$ level is abnormal; e.g., by comparing the mean $CO_2$ level to a predetermined range of acceptable $CO_2$ levels. If the $CO_2$ level is determined to be within the predetermined range of acceptable $CO_2$ levels (i.e., normocapnia), then the collected $StO_2$ and MAP data may be used in a determination of the subject's autoregulation state. If the $CO_2$ level is determined to be outside of the predetermined range of acceptable $CO_2$ levels (i.e., hypercapnia or hypocapnia), then the subject's $CO_2$ level may be a confounding factor, and the collected $StO_2$ and MAP data may not be used in a determination of the subject's autoregulation state (or any autoregulation data produced using the collected $StO_2$ and MAP data may be flagged or not produced, etc.).

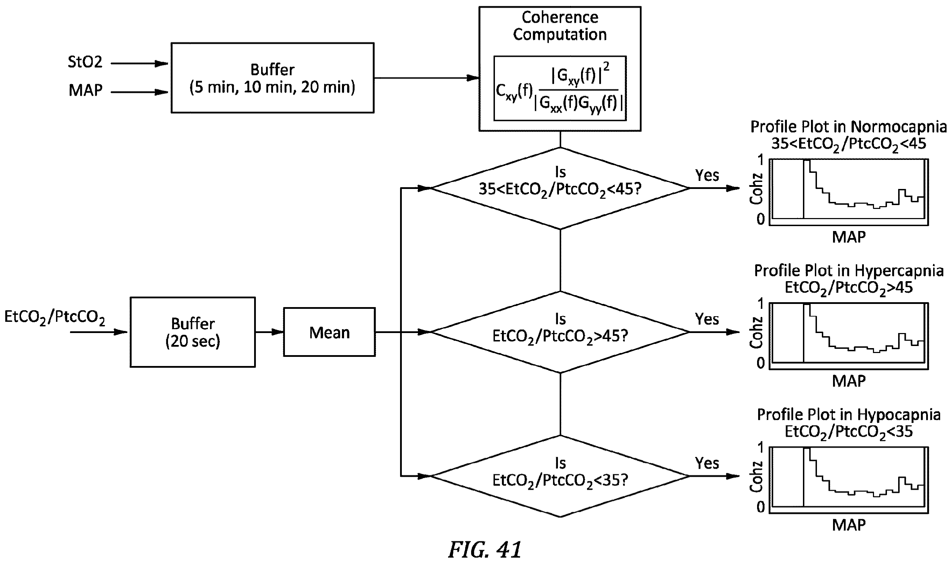

In a second example diagrammatically shown in FIG. 41, an analysis methodology similar to that described above with respect to FIG. 40 is performed. Here again, NIRS index data (e.g., $StO_2$), MAP data, and blood $CO_2$ data are collected over a predetermined period of time, the collected $StO_2$ and MAP data may be organized within a time domain and processed using a correlation technique, or organized to produce COHZ values in a manner such as described above (collectively referred to as "COHZ" values below to simplify the description), and the collected $CO_2$ level data may be mathematically evaluated to determine a mean value. In this example, COHZ values associated with $CO_2$ level data within a normocapnia range may be used to produce first autoregulation data, COHZ values associated with $CO_2$ level data within a hypercapnia range may be used to produce second autoregulation data (e.g., a plot of COHZ values versus MAP), and COHZ values associated with $CO_2$ level data within a hypocapnia range may be used to produce third autoregulation data. As shown in FIG. 41, the autoregulation data for each of these groups may be organized as a plot of COHZ values versus MAP, but the present disclosure is not limited to this type of data organization; e.g., the autoregulation data could be organized in look-up tables, etc. Autoregulation data produced and organized as shown in FIG. 41 provides unique, detailed information to a clinician that can greatly facilitate autoregulation state evaluation.

In some instances, a NIRS index value (e.g., $StO_2$) associated with a subject's extracerebral blood flow (e.g., within the subject's scalp) may differ from the same NIRS index value associated with a subject's cerebral blood flow, and the aforesaid difference may be independent of the subject's autoregulation. In such an instance, the extracerebral blood flow may be a confounding factor. For example, a cerebral oximeter measured $StO_2$ value may be skewed if the $StO_2$ level within the subject's scalp blood flow is meaningfully different from the $StO_2$ level of the subject's cerebral blood flow. A difference in extracerebral versus brain tissue $StO_2$ levels may occur, for example, if a vasodilator or vasoconstrictor has been administered that affects cerebral and peripheral circulatory vasculature differently. Under these circumstances, the difference in $StO_2$ values between scalp blood flow and cerebral blood flow could give an erroneous indication of a normal autoregulation state, when in fact the $StO_2$ value of the subject's extracerebral blood flow is independent of the subject's autoregulation state. Embodiments of the present disclosure may be configured to determine relative differences, changes and/or trending of a subject's extracerebral $StO_2$ and cerebral blood flow, and based on that determination subsequently determine the extracerebral blood flow is acting as a confounding factor, and therefore whether the collected physiological data (e.g., NIRS indices, etc.) should be used in the determination of autoregulation state.

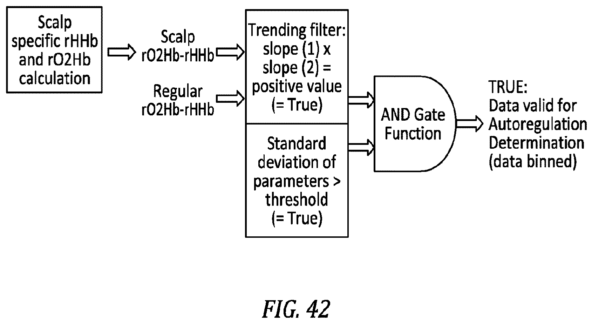

FIG. 42 illustrates an exemplary functional diagram of how NIRS index trend data may be used to determine whether extracerebral blood flow may be acting as a confounding factor, and subsequently whether the NIRS index data should be used in the determination of autoregulation state. The present disclosure may be configured to determine whether extracerebral blood flow may be acting as a confounding factor by sensing one or more NIRS indices (e.g., rO2Hb, rHHb) over a predetermined period of time. In this example, the sensing of the NIRS indices (e.g., rO2Hb, rHHb) includes determining those NIRS indices in a scalp specific manner and in a conventional tissue oximetry manner. For example, a tissue oximeter (e.g., a ForeSight Elite® tissue oximeter produced by Edwards Lifesciences Corporation) having one or more sensors, each with a light source, a near light detector, and one or more far detectors may be attached to a subject's forehead. NIRS light collected by the near detector represents light that has interrogated shallow depth tissue (e.g., extracerebral tissue) and NIRS light collected by the one or more far detectors represents light that has interrogated both shallow and deep depth tissue (e.g., cerebral tissue). The collected light may be processed to determine NIRS index values (e.g., rO2Hb, rHHb) for the shallow depth tissue and the shallow and deep depth tissue. As indicated above, U.S. Patent Nos. 7,072,701; 8,078,250; 8,396,526; and 8,965,472; and 10,117,610, each of which is assigned to Edwards Lifesciences Corporation, disclose non-limiting examples of non-invasive NIRS tissue oximeters that may be used to determine NIRS index values (e.g., $rHbO_2$, rHb) for shallow depth tissue and shallow and deep depth tissue. The present disclosure is not limited to these tissue oximeter examples, however.

In a manner similar to that described above, the NIRS indices (e.g., rO2Hb, rHHb) sensed over a predetermined period of time may be processed to create trend data for the respective NIRS index; e.g., the respective NIRS index values (e.g., rO2Hb, rHHb, rO2Hb - rHHb, etc.) collected periodically over a period of time may be mathematically evaluated using a regression line fit analysis and the determined slopes of the respective NIRS index regression lines may then be comparatively analyzed. Also similar to the processes described above, embodiments of the present disclosure evaluating extracerebral blood flow as a possible confounding factor may also evaluate the magnitude of change of the factor being considered; i.e., if the relevant parameters being considered evidence a change, but that change is minimal (e.g., below a threshold amount), then the confounding factor is likely to be not clinically relevant. The product of the regression line slopes and the standard deviation values may be input to a logic gate (e.g., an "AND gate") or other trending filter. If the product of the regression line slopes indicates the compared NIRS indices (e.g., rO2Hb, rHHb, rO2Hb - rHHb, etc. values for scalp alone and for shallow and deep tissue) are trending in the same manner (e.g., a positive value), and the standard deviation values indicate trends of sufficient magnitude, then both represent a "true" input to the logic gate and the NIRS index data is determined to be useful in a determination of the subject's autoregulation state; i.e., the extracerebral blood flow is not acting as a confounding factor. Conversely, if the product of the regression line slopes does not indicate that the NIRS indices are trending in the same manner (i.e., a negative value), that would be a "false" input to the logic gate - an indicator of extracerebral blood flow as a confounding factor, and the NIRS index data may not be used in a determination of the subject's autoregulation state. Here again, the standard deviation values can be used to evaluate whether the trends are of sufficient magnitude to warrant not using the data (or flagging the results, etc.) in a determination of the subject's autoregulation state.

As indicated above, the functionality described herein may be implemented, for example, in hardware, software tangibly embodied in a computer-readable medium, firmware, or any combination thereof. In some embodiments, at least a portion of the functionality described herein may be implemented in one or more computer programs. Each such computer program may be implemented in a computer program product tangibly embodied in non-transitory signals in a machine-readable storage device for execution by a computer processor. Method steps of the present disclosure may be performed by a computer processor executing a program tangibly embodied on a computer-readable medium to perform functions of the present disclosure by operating on input and generating output. Each computer program within the scope of the present claims below may be implemented in any programming language, such as assembly language, machine language, a high-level procedural programming language, or an object-oriented programming language. The programming language may, for example, be a compiled or interpreted programming language.

While the principles of the disclosure have been described above in connection with specific apparatuses and methods, it is to be clearly understood that this description is made only by way of example and not as limitation on the scope of the disclosure. Specific details are given in the above description to provide a thorough understanding of the embodiments. However, it is understood that the embodiments may be practiced without these specific details.

It is noted that the embodiments may be described as a process which is depicted as a flowchart, a flow diagram, a block diagram, etc. Although any one of these structures may describe the operations as a sequential process, many of the operations can be performed in parallel or concurrently. In addition, the order of the operations may be rearranged. A process may correspond to a method, a function, a procedure, a subroutine, a subprogram, etc.

The singular forms "a," "an," and "the" refer to one or more than one, unless the context clearly dictates otherwise. For example, the term "comprising a specimen" includes single or plural specimens and is considered equivalent to the phrase "comprising at least one specimen." The term "or" refers to a single element of stated alternative elements or a combination of two or more elements unless the context clearly indicates otherwise. As used herein, "comprises" means "includes." Thus, "comprising A or B," means "including A or B, or A and B," without excluding additional elements.

It is noted that various connections are set forth between elements in the present description and drawings (the contents of which are included in this disclosure by way of reference). It is noted that these connections are general and, unless specified otherwise, may be direct or indirect and that this specification is not intended to be limiting in this respect. Any reference to attached, fixed, connected or the like may include permanent, removable, temporary, partial, full and/or any other possible attachment option.

No element, component, or method step in the present disclosure is intended to be dedicated to the public regardless of whether the element, component, or method step is explicitly recited in the claims. No claim element herein is to be construed under the provisions of 35 U.S.C. 112(f) unless the element is expressly recited using the phrase "means for." As used herein, the terms "comprises", "comprising", or any other variation thereof, are intended to cover a non-exclusive inclusion, such that a process, method, article, or apparatus that comprises a list of elements does not include only those elements but may include other elements not expressly listed or inherent to such process, method, article, or apparatus.

While various inventive aspects, concepts and features of the disclosures may be described and illustrated herein as embodied in combination in the exemplary embodiments, these various aspects, concepts, and features may be used in many alternative embodiments, either individually or in various combinations and sub-combinations thereof. Unless expressly excluded herein all such combinations and sub-combinations are intended to be within the scope of the present application. Still further, while various alternative embodiments as to the various aspects, concepts, and features of the disclosures--such as alternative materials, structures, configurations, methods, devices, and components, and so on--may be described herein, such descriptions are not intended to be a complete or exhaustive list of available alternative embodiments, whether presently known or later developed. Those skilled in the art may readily adopt one or more of the inventive aspects, concepts, or features into additional embodiments and uses within the scope of the present application even if such embodiments are not expressly disclosed herein. For example, in the exemplary embodiments described above within the Detailed Description portion of the present specification, elements may be described as individual units and shown as independent of one another to facilitate the description. In alternative embodiments, such elements may be configured as combined elements.

Additionally, even though some features, concepts, or aspects of the disclosures may be described herein as being a preferred arrangement or method, such description is not intended to suggest that such feature is required or necessary unless expressly so stated. Still further, exemplary or representative values and ranges may be included to assist in understanding the present application, however, such values and ranges are not to be construed in a limiting sense and are intended to be critical values or ranges only if so expressly stated.

Examples are set out in the following list of numbered clauses:

1. A method for determining a subject's autoregulation function state, comprising:

continuously sensing a tissue region of a subject with a tissue oximeter, the sensing producing first signals representative of at least one tissue oxygenation parameter during a period of time;
continuously measuring a blood pressure level of the subject using a blood pressure sensing device during the period of time, the measuring producing second signals representative of the blood pressure of the subject during the period of time;
determining a presence or an absence of a confounding factor that affects the sensed at least one tissue oxygenation parameter in a manner independent of an autoregulation function of the subject, the determination using the first signals; and
using the first signals and the second signals to determine an autoregulation function state of the subject when the absence of the confounding factor is determined.

2. The method of clause 1, wherein the step of determining said presence or said absence of the confounding factor further includes determining whether the confounding factor has affected the measured blood pressure level of the subject in a manner independent of the autoregulation function of the subject using the second signals.

3. The method of clause 2, wherein the step of determining said presence or said absence of the confounding factor utilizes a tissue oxygenation parameter trend data based on the first signals, and a blood pressure level trend data based on the second signals.

4. The method of clause 1, further comprising determining a heart rate of the subject during the period of time, and producing third signals representative of the subject's heart rate during the period of time; and
wherein the step of determining said presence or said absence of the confounding factor further includes determining whether the confounding factor has affected the heart rate of the subject in a manner independent of the autoregulation function of the subject using the third signals.

5. The method of clause 4, wherein the step of determining said presence or said absence of the confounding factor utilizes a tissue oxygenation parameter trend data based on the first signals, and a heart rate trend data based on the third signals.

6. The method of clause 1, wherein the step of determining said presence or said absence of the confounding factor further includes evaluating the first signals using a magnitude of change filter.

7. The method of clause 1, wherein the at least one tissue oxygenation parameter includes one or more of tissue oxygen saturation ($StO_2$), total hemoglobin concentration per volume of tissue (THb), relative total hemoglobin concentration per volume of tissue (rTHb), differential changes in oxyhemoglobin (O2Hb) and deoxyhemoglobin (HHb), or HbD.

8. The method of clause 1, wherein the step of determining said presence or said absence of the confounding factor further includes determining a blood carbon dioxide ($CO_2$) level of the subject.

9. The method of clause 1, wherein the at least one tissue oxygenation parameter includes a first tissue oxygenation parameter and a second oxygenation parameter; and

wherein the first signals produced from the sensing include a first subset of first signals representative of the first tissue oxygenation parameter, and a second subset of first signals representative of the second tissue oxygenation parameter; and
the step of determining said presence or said absence of the confounding factor utilizes the first subset of first signals and the second subset of first signals.

10. The method of clause 9, wherein the first tissue oxygenation parameter is one of $StO_2$, THb, rTHb, differential changes in oxyhemoglobin (O2Hb) and deoxyhemoglobin (HHb), or HbD, and the second tissue oxygenation parameter is another of $StO_2$, THb, rTHb, differential changes in oxyhemoglobin (O2Hb) and deoxyhemoglobin (HHb), or HbD.

11. The method of clause 9, wherein the step of determining said presence or said absence of the confounding factor utilizes a first tissue oxygenation parameter trend data based on the first subset of first signals, and a second tissue oxygenation parameter trend data based on the second subset of first signals.

12. The method of clause 1, wherein the tissue oximeter is a near-infrared spectroscopy type tissue oximeter.

13. The method of clause 1, wherein the tissue being continuously sensed is brain tissue, and the autoregulation function state determined in the absence of the confounding factor is a brain autoregulation function state of the subject.

14. The method of clause 13, wherein the step of determining said presence or said absence of the confounding factor further includes evaluating the first signals to determine extracerebral blood flow as the confounding factor.

15. The method of clause 14, wherein the step of continuously sensing the tissue region of the subject with the tissue oximeter, includes using one or more sensors in communication with the tissue oximeter, the one or more sensors each having at least one light source, at least one near detector located a first distance from the at least one light source, and at least one far detector located a second distance from the at least one light source, where the second distance is greater than the first distance.

16. An apparatus for determining a subject's autoregulation function state, comprising:

a near infra-red spectroscopy (NIRS) tissue oximeter, configured to continuously sense a tissue region of the subject during a period of time, and to produce first signals representative of at least one tissue oxygenation parameter during the period of time;
a blood pressure sensing device, configured to continuously measure a blood pressure level of the subject during the period of time, and to produce second signals representative of the blood pressure of the subject during the period of time; and
a controller in communication with the NIRS tissue oximeter and the blood pressure sensing device, the controller including at least one processor and a memory device configured to store instructions, the stored instructions when executed cause the controller to:

control the NIRS tissue oximeter to continuously sense a tissue region of the subject during a period of time, and to produce first signals representative of at least one tissue oxygenation parameter sensed within the tissue region during the period of time;
control the blood pressure sensing device to continuously measure a blood pressure level of the subject during the period of time, and to produce second signals representative of the blood pressure of the subject during the period of time;
determine a presence or an absence of a confounding factor using the first signals, the confounding factor operable to affect the sensed at least one tissue oxygenation parameter in a manner independent of an autoregulation function of the subject; and
using the first signals and the second signals, determine an autoregulation function state of the subject when the absence of the confounding factor is determined.

17. The apparatus of clause 16, wherein the stored instructions when executed cause the at least one processor to determine the autoregulation function state of the subject without using the first signals and the second signals when the presence of the confounding factor is determined.

18. The apparatus of clause 16, wherein the stored instructions when executed cause the at least one processor to determine the autoregulation function state of the subject using the first signals and the second signals when the presence of the confounding factor is determined, and to flag the autoregulation function state.

19. The apparatus of clause 16, wherein the stored instructions when executed cause the controller to determine whether the confounding factor has affected the measured blood pressure level of the subject in a manner independent of the autoregulation function of the subject using the second signals.

20. The apparatus of clause 19, wherein the stored instructions when executed cause the controller to determine a tissue oxygenation parameter trend data based on the first signals and to determine a blood pressure level trend data based on the second signals, and the determination of said presence or said absence of the confounding factor utilizes the tissue oxygenation parameter trend data and the blood pressure level trend data.

21. The apparatus of clause 16, wherein the stored instructions when executed cause the controller to determine a heart rate of the subject during the period of time, and to produce third signals representative of the subject's heart rate during the period of time; and
the stored instructions when executed cause the controller to determine whether the confounding factor has affected the heart rate of the subject in a manner independent of the autoregulation function of the subject using the third signals.

22. The apparatus of clause 21, wherein the stored instructions when executed cause the controller to determine a tissue oxygenation parameter trend data based on the first signals, and a heart rate trend data based on the third signals, and the determination of said presence or said absence of the confounding factor utilizes the tissue oxygenation parameter trend data and the heart rate trend data.

23. The apparatus of clause 16, wherein the stored instructions when executed cause the controller to evaluate the first signals using a magnitude of change filter.

24. The apparatus of clause 16, wherein the at least one tissue oxygenation parameter includes one or more of tissue oxygen saturation ($StO_2$), total hemoglobin concentration per volume of tissue (THb), relative total hemoglobin concentration per volume of tissue (rTHb), differential changes in oxyhemoglobin (O2Hb) and deoxyhemoglobin (HHb), or HbD.

25. The apparatus of clause 16 further comprising a CO2 sensor configured to sense a blood carbon dioxide ($CO_2$) level of the subject, and the instructions when executed cause the controller to determine a blood carbon dioxide ($CO_2$) level of the subject using the CO2 sensor.

26. The apparatus of clause 16, wherein the at least one tissue oxygenation parameter includes a first tissue oxygenation parameter and a second oxygenation parameter; and

wherein the first signals produced from the sensing include a first subset of first signals representative of the first tissue oxygenation parameter, and a second subset of first signals representative of the second tissue oxygenation parameter; and
the determination of said presence or said absence of the confounding factor utilizes the first subset of first signals and the second subset of first signals.

27. The apparatus of clause 26, wherein the first tissue oxygenation parameter is one of $StO_2$, THb, rTHb, differential changes in O2Hb and HHb, or HbD, and the second tissue oxygenation parameter is another of $StO_2$, THb, rTHb, differential changes in O2Hb and HHb, or HbD.

28. The apparatus of clause 26, wherein the determination of said presence or said absence of the confounding factor utilizes a first tissue oxygenation parameter trend data based on the first subset of first signals, and a second tissue oxygenation parameter trend data based on the second subset of first signals.

29. The apparatus of clause 16, wherein the tissue oximeter is configured to sense extracerebral tissue and cerebral tissue; and
the stored instructions when executed cause the tissue oximeter to continuous sense extracerebral tissue and brain tissue, and the autoregulation function state determined in the absence of the confounding factor is a brain autoregulation function state of the subject.

30. The apparatus of clause 29, wherein the stored instructions when executed cause the controller to determine extracerebral blood flow as the confounding factor.

31. The apparatus of clause 29, wherein the tissue oximeter includes one or more sensors each having at least one light source, at least one near detector located a first distance from the at least one light source, and at least one far detector located a second distance from the at least one light source, where the second distance is greater than the first distance.

32. A method for determining at least one of a lower limit of autoregulation (LLA) or an upper limit of autoregulation (ULA) of a subject's autoregulation function state, comprising:

continuously sensing a tissue region of a subject with a tissue oximeter, the sensing producing first signals representative of at least one tissue oxygenation parameter during a period of time;
continuously measuring a blood pressure level of the subject using a blood pressure sensing device during the period of time, the measuring producing second signals representative of the blood pressure of the subject during the period of time;
determining autoregulation data as a function of subject blood pressure using the first signals representative of at least one tissue oxygenation parameter and the second signals representative of the blood pressure of the subject during the period of time; and
determining at least one of an LLA or a ULA of the subject's autoregulation function state.

33. The method of clause 32, wherein the step of determining at least one of an LLA or a ULA of the subject's autoregulation function state includes fitting a curve to the autoregulation data as a function of subject blood pressure using an algorithm.

34. The method of clause 33, wherein the step of fitting the curve to the autoregulation data includes determining an algorithmic model of the curve; and
wherein the step of determining at least one of said LLA or said ULA of the subject's autoregulation function state using the fitted curve includes determining an inflection point using the algorithmic model of the curve.

35. The method of clause 34, wherein the determining said inflection point includes determining a first derivative of the curve.

36. The method of clause 35, wherein the determining said inflection point uses at least some of said autoregulation data.

37. The method of clause 34, wherein the determining said inflection point includes determining a second derivative of the curve.

38. The method of clause 33, wherein the step of determining at least one of said LLA or said ULA of the subject's autoregulation function state using the fitted curve includes utilizing the first derivative of the fitted curve, the second derivative of the fitted curve, or the absolute value of the fitted curve, or any combination thereof.

39. The method of clause 33, wherein the step of determining at least one of said LLA or said ULA of the subject's autoregulation function state using the fitted curve includes utilizing an elbow point method.

40. The method of clause 32, wherein the step of determining autoregulation data as a function of subject blood pressure includes binning the autoregulation data determined for the period of time as a function of a plurality of incremental blood pressure bins;
wherein the method further comprises determining a confidence value for the autoregulation data in each incremental blood pressure bin.

41. The method of clause 40, further comprising visually displaying the binned autoregulation data as a function of said incremental blood pressure bins.

42. The methods of clause 40, further comprising visually displaying the confidence value for the autoregulation data in each incremental blood pressure bin on top of the binned autoregulation data.

43. The method of clause 40, wherein the step of determining said confidence value for the autoregulation data in each incremental blood pressure bin includes determining first order statistical information for the autoregulation data in each respective incremental blood pressure bin, and determining the respective confidence value using the determined first order statistical information.

44. The method of clause 33, wherein the step of determining autoregulation data as a function of subject blood pressure includes binning the autoregulation data determined for the period of time as a function of a plurality of incremental blood pressure bins; and

wherein the method further comprises determining a confidence value for the autoregulation data in each incremental blood pressure bin; and
wherein the step of fitting the curve to the autoregulation data includes evaluating the autoregulation data in each incremental blood pressure bin for inclusion in the curve fitting based on the determined confidence value for the autoregulation data in that respective incremental blood pressure bin.

45. An apparatus for determining a subject's autoregulation function state, comprising:

a near infra-red spectroscopy (NIRS) tissue oximeter, configured to continuously sense a tissue region of the subject during a period of time, and to produce first signals representative of at least one tissue oxygenation parameter during the period of time;
a blood pressure sensing device, configured to continuously measure a blood pressure level of the subject during the period of time, and to produce second signals representative of the blood pressure of the subject during the period of time; and

a controller in communication with the NIRS tissue oximeter and the blood pressure sensing device, the controller including at least one processor and a memory device configured to store instructions, the stored instructions when executed cause the controller to:

control the NIRS tissue oximeter to continuously sense a tissue region of the subject during a period of time, and to produce first signals representative of at least one tissue oxygenation parameter sensed within the tissue region during the period of time;

control the blood pressure sensing device to continuously measure a blood pressure level of the subject during the period of time, and to produce second signals representative of the blood pressure of the subject during the period of time;

determine autoregulation data using the first signals and the second signals; and

determine at least one of a lower limit of autoregulation (LLA) or an upper limit of autoregulation (ULA) of the subject's autoregulation function state.

46. The apparatus of clause 45, wherein the instructions when executed include causing the controller to fit a curve to the autoregulation data as a function of subject blood pressure using an algorithm.

47. The apparatus of clause 46, wherein the instructions when executed cause the controller to determine an algorithmic model of the curve, and use the algorithmic model to fit the curve to the autoregulation data; and determine an inflection point using the algorithmic model of the curve in the determination of the at least one of the LLA or the ULA of the subject's autoregulation function state.

48. The apparatus of clause 46, wherein the instructions when executed that cause the controller to determine the autoregulation data, also cause the controller to bin the autoregulation data determined for the period of time as a function of a plurality of incremental blood pressure bins, and determine a confidence value for the autoregulation data in each incremental blood pressure bin.

49. The apparatus of clause 48, wherein the instructions when executed cause the controller to evaluate the autoregulation data in each incremental blood pressure bin for inclusion in the curve fitting based on the determined confidence value for the autoregulation data in that respective incremental blood pressure bin.

50. A non-transitory computer-readable medium containing computer program instructions, wherein the computer program instructions are executable by the at least one computer processor to perform a method of non-invasively determining a tissue arterial oxygen saturation value of a tissue body, the method comprising:

controlling a light source to transmit at least a first wavelength and a second wavelength of near-infrared light into a tissue body, the first wavelength different from the second wavelength;

controlling at least one light detector to sense the tissue body for the near-infrared light, and producing signals representative of the sensed near-infrared light;

determining an AC component of a first tissue oxygen parameter using the signals;

determining an AC component of a second tissue oxygen parameter using the signals; and

determining a tissue arterial oxygen saturation value of a tissue body using the determined AC component of the first tissue oxygen parameter and the determined AC component of the second tissue oxygen parameter.

50. A non-transitory computer-readable medium containing computer program instructions, wherein the computer program instructions are executable by the at least one computer processor to perform a method of determining a subject's autoregulation function state, the method comprising:

controlling a tissue oximeter to continuously sense a tissue region of a subject, the sensing producing first signals representative of at least one tissue oxygenation parameter during a period of time;

controlling a blood pressure sensing device to continuously measure a blood pressure level of the subject, the measuring producing second signals representative of the blood pressure of the subject during the period of time;

determining a presence or an absence of a confounding factor that affects the sensed at least one tissue oxygenation parameter in a manner independent of an autoregulation function of the subject, the determination using the first signals; and

using the first signals and the second signals to determine an autoregulation function state of the subject when the absence of the confounding factor is determined.

51. A non-transitory computer-readable medium containing computer program instructions, wherein the computer program instructions are executable by the at least one computer processor to perform a method of determining at least one of a lower limit of autoregulation (LLA) or an upper limit of autoregulation (ULA) of a subject's autoregulation function state, the method comprising:

controlling a tissue oximeter to continuously sense a tissue region of a subject, the sensing producing first signals representative of at least one tissue oxygenation parameter during a period of time; controlling a blood pressure sensing device to continuously measure a blood pressure level of the subject using a blood pressure sensing device during the period of time, the measuring producing second signals representative of the blood pressure of the subject during the period of time; determining autoregulation data as a function of subject blood pressure using the first signals representative of at least one tissue oxygenation parameter and the second signals representative of the blood pressure of the subject during the period of time; and determining at least one of an LLA or a ULA of the subject's autoregulation function state.

## Claims

1. A method for determining at least one of a lower limit of autoregulation (LLA) or an upper limit of autoregulation (ULA) of a subject's autoregulation function state, comprising:

continuously sensing a tissue region of a subject with a tissue oximeter, the sensing producing first signals representative of at least one tissue oxygenation parameter during a period of time; continuously measuring a blood pressure level of the subject using a blood pressure sensing device during the period of time, the measuring producing second signals representative of the blood pressure of the subject during the period of time; determining autoregulation data as a function of subject blood pressure using the first signals representative of at least one tissue oxygenation parameter and the second signals representative of the blood pressure of the subject during the period of time; and determining at least one of an LLA or a ULA of the subject's autoregulation function state.

2. The method of claim 1, wherein the step of determining at least one of an LLA or a ULA of the subject's autoregulation function state includes fitting a curve to the autoregulation data as a function of subject blood pressure using an algorithm.

3. The method of claim 2, wherein the step of fitting the curve to the autoregulation data includes determining an algorithmic model of the curve; and wherein the step of determining at least one of said LLA or said ULA of the subject's autoregulation function state using the fitted curve includes determining an inflection point using the algorithmic model of the curve.

4. The method of claim 3, wherein the determining said inflection point includes:

a) determining a first derivative of the curve, optionally wherein the determining said inflection point uses at least some of said autoregulation data; or
b) determining a second derivative of the curve.

5. The method of claim 2, wherein the step of determining at least one of said LLA or said ULA of the subject's autoregulation function state using the fitted curve includes:

a) utilizing the first derivative of the fitted curve, the second derivative of the fitted curve, or the absolute value of the fitted curve, or any combination thereof; or
b) utilizing an elbow point method.

6. The method of any preceding claim, wherein the step of determining autoregulation data as a function of subject blood pressure includes binning the autoregulation data determined for the period of time as a function of a plurality of incremental blood pressure bins;

wherein the method further comprises determining a confidence value for the autoregulation data in each incremental blood pressure bin.

7. The method of claim 6, further comprising visually displaying:

   a) the binned autoregulation data as a function of said incremental blood pressure bins; or
   b) the confidence value for the autoregulation data in each incremental blood pressure bin on top of the binned autoregulation data.

8. The method of claim 6 or 7, wherein the step of determining said confidence value for the autoregulation data in each incremental blood pressure bin includes determining first order statistical information for the autoregulation data in each respective incremental blood pressure bin, and determining the respective confidence value using the determined first order statistical information.

9. The method of claim 2, wherein the step of determining autoregulation data as a function of subject blood pressure includes binning the autoregulation data determined for the period of time as a function of a plurality of incremental blood pressure bins; and

   wherein the method further comprises determining a confidence value for the autoregulation data in each incremental blood pressure bin; and
   wherein the step of fitting the curve to the autoregulation data includes evaluating the autoregulation data in each incremental blood pressure bin for inclusion in the curve fitting based on the determined confidence value for the autoregulation data in that respective incremental blood pressure bin.

10. An apparatus for determining a subject's autoregulation function state, comprising:

    a near infra-red spectroscopy (NIRS) tissue oximeter, configured to continuously sense a tissue region of the subject during a period of time, and to produce first signals representative of at least one tissue oxygenation parameter during the period of time;
    a blood pressure sensing device, configured to continuously measure a blood pressure level of the subject during the period of time, and to produce second signals representative of the blood pressure of the subject during the period of time; and
    a controller in communication with the NIRS tissue oximeter and the blood pressure sensing device, the controller including at least one processor and a memory device configured to store instructions, the stored instructions when executed cause the controller to:

       control the NIRS tissue oximeter to continuously sense a tissue region of the subject during a period of time, and to produce first signals representative of at least one tissue oxygenation parameter sensed within the tissue region during the period of time;
       control the blood pressure sensing device to continuously measure a blood pressure level of the subject during the period of time, and to produce second signals representative of the blood pressure of the subject during the period of time;
       determine autoregulation data using the first signals and the second signals; and
       determine at least one of a lower limit of autoregulation (LLA) or an upper limit of autoregulation (ULA) of the subject's autoregulation function state.

11. The apparatus of claim 10, wherein the instructions when executed include causing the controller to fit a curve to the autoregulation data as a function of subject blood pressure using an algorithm.

12. The apparatus of claim 11, wherein the instructions when executed cause the controller to determine an algorithmic model of the curve, and use the algorithmic model to fit the curve to the autoregulation data; and
    determine an inflection point using the algorithmic model of the curve in the determination of the at least one of the LLA or the ULA of the subject's autoregulation function state.

13. The apparatus of claim 11, wherein the instructions when executed that cause the controller to determine the autoregulation data, also cause the controller to bin the autoregulation data determined for the period of time as a function of a plurality of incremental blood pressure bins, and determine a confidence value for the autoregulation data in each incremental blood pressure bin.

**14.** The apparatus of claim 13, wherein the instructions when executed cause the controller to evaluate the autoregulation data in each incremental blood pressure bin for inclusion in the curve fitting based on the determined confidence value for the autoregulation data in that respective incremental blood pressure bin.

**15.** A non-transitory computer-readable medium containing computer program instructions, wherein the computer program instructions are executable by the at least one computer processor to perform a method of determining at least one of a lower limit of autoregulation (LLA) or an upper limit of autoregulation (ULA) of a subject's autoregulation function state, the method comprising:

controlling a tissue oximeter to continuously sense a tissue region of a subject, the sensing producing first signals representative of at least one tissue oxygenation parameter during a period of time;
controlling a blood pressure sensing device to continuously measure a blood pressure level of the subject using a blood pressure sensing device during the period of time, the measuring producing second signals representative of the blood pressure of the subject during the period of time;
determining autoregulation data as a function of subject blood pressure using the first signals representative of at least one tissue oxygenation parameter and the second signals representative of the blood pressure of the subject during the period of time; and
determining at least one of an LLA or a ULA of the subject's autoregulation function state.

**FIG. 1**
(PRIOR ART)

**FIG. 2**
(PRIOR ART)

**FIG. 3**
(PRIOR ART)

**FIG. 4A**

*FIG. 4B*

*FIG. 5*

StO2

38

AR Index

MBP range

COHZ
NIRS

*FIG. 6*

*FIG. 7*

*FIG. 8*

EP 4 678 093 A2

COHZ FREQUENCY BAND #1:
(e.g., 0.00333-0.05 Hz)
5 minute Sampling Window

COHZ FREQUENCY BAND #2:
(e.g., 0.00166-0.05 Hz)
10 minute Sampling Window

COHZ FREQUENCY BAND #3:
(e.g., 0.000833-0.05 Hz)
20 minute Sampling Window

COHZ FREQUENCY BAND #4:
(e.g., 0.05-0.15 Hz)
5 minute Sampling Window

COHZ FREQUENCY BAND #5
(e.g., 0.08-0.12 Hz) - Mayer Wave
5 minute Sampling Window

COHZ Peak
Detector

36

AR Index:
Determined
MAX COHZ

*FIG. 9*

COHZ FREQUENCY
BAND #1:
(e.g., 0.00333-0.05 Hz)
5 minute Sampling Window

COHZ FREQUENCY BAND #5:
(e.g., 0.08-0.12 Hz)
- Mayer Wave
5 minute Sampling Window

COHZ FREQUENCY BAND #2:
(e.g., 0.00166-0.05 Hz)
10 minute Sampling Window

COHZ FREQUENCY BAND #3:
(e.g., 0.000833-0.05 Hz)
20 minute Sampling Window

COHZ FREQUENCY BAND #4:
(e.g., 0.05-0.15 Hz)
5 minute Sampling Window

0.000833 Hz                    0.05 Hz                    0.15 Hz

FREQUENCY

*FIG. 10*

COHZ FREQUENCY
BAND #4:
(e.g., 0.05-0.15 Hz)
5 minute Sampling
Window

COHZ FREQUENCY
BAND #5:
(e.g., 0.08-0.12 Hz)
Mayer Wave
5 minute Sampling
Window

COHZ FREQUENCY
BAND #1:
(e.g., 0.00333-0.05 Hz)
5 minute Sampling
Window

COHZ FREQUENCY BAND #2:
(e.g., 0.00166-0.05 Hz)
10 minute Sampling Window

COHZ FREQUENCY BAND #3:
(e.g., 0.000833-0.05 Hz)
20 minute Sampling Window

$T_{-20mins}$              $T_{-10mins}$    $T_{-5mins}$        $T_{PRESENT}$

TIME

*FIG. 11*

FIG. 12

FIG. 12A

*FIG. 13*

*FIG. 14*

*FIG. 15*

38

40 — AR COHZ 1.0 ... 0.0 / BP Range / 20 ... 140

42 — NIRS 100.0 ... 0.0 / BP Range / 20 ... 140

44 — Real time BP/NIRS/COHZ / BP / NIRS / COHZ / Amplitude 120 ... 10 / Time / 1100 ... 2100

*FIG. 16*

NLR SIGMOIDAL CURVE MODEL FOR AR Profile

*FIG. 17*

AR Curve generated from sigmoidal function fit for AR Profile Plot

FIG. 18

FIG. 19

*FIG. 20*

*FIG. 21*

*FIG. 22*

FIG. 23

*FIG. 24A*

*FIG. 24B*

*FIG. 24C*

*FIG. 24D*

*FIG. 24E*

*FIG. 25*

COLLECT PHYSIOLOGICAL DATA (e.g., NIRS INDICES, BLOOD PRESSURE DATA, CO2, ETC.

EVALUATE PHYSIOLOGICAL DATA TO DETERMINE PRESENCE OR ABSENCE OF CONFOUNDING FACTOR

YES | CONFOUNDING FACTOR PRESENT? | NO

TAKE ACTION: e.g.,
- DO NOT USE PHYSIOLOGICAL DATA IN AUTOREGULATION DETERMINATION;

- DO NOT PRODUCE AUTOREGULATION RESULTS

- FLAG AUTOREGULATION RESULTS, ETC.

DETERMINE OR MEASURE SUBJECT'S AUTOREGULATION STATE

*FIG. 26*

*FIG. 27*

*FIG. 28*

| Case | BP trend | StO2 trend | rTHb trend | CAI Data Valid | CAI Interpretation | AR Interpretation |
|---|---|---|---|---|---|---|
| 1 | Incr | Incr | Incr | Yes | BP & StO2 changes likely related (high CAI) | Change in flow indicated by StO2 & rTHb change related to BP (poor AR) |
| 2 | Decr | Decr | Decr | Yes | BP & StO2 changes likely related (high CAI) | Change in flow indicated by StO2 & rTHb change related to BP (poor AR) |
| 3 | Incr | N/C | N/C | Yes | BP & StO2 changes likely related, StO2 not likely affected by other physiological process (low CAI) | No change in flow indicated by StO2 & rTHb constant related to BP (poor AR) |
| 4 | Decr | N/C | N/C | Yes | BP & StO2 changes likely related, StO2 not likely affected by other physiological process (low CAI) | No change in flow indicated by StO2 & rTHb constant related to BP (poor AR) |
| 5 | Incr | Decr | any | No | BP & StO2 changes not likely related or relationship cannot be determined, StO2 possibly affected by other physiological process | Polarity filter: StO2 relationship to BP and AR unknown |
| 6 | Decr | Incr | any | No | BP & StO2 changes not likely related or relationship cannot be determined, StO2 possibly affected by other physiological process | Polarity filter: StO2 relationship to BP and AR unknown |
| 7 | Incr | any | any | No | BP & StO2 changes relationship cannot be determined if BP constant | Flat filter: BP needs to change for AR determination |
| 8 | Decr | Decr | Incr | No | Indication of venous congestion; BP & StO2 changes not related | Physiology filter: venous congestion confounder to AR determination |
| 9 | Incr | Incr | Decr | No | Indication of resolving venous congestion; BP & StO2 changes not related | Physiology filter: resolving venous congestion confounder to AR determination |
| 10 | Decr | Decr | N/C | No | BP & StO2 changes not likely related or relationship cannot be determined, StO2 possibly affected by other physiological process (hypoxia) | Physiology filter: hypoxia event confounder to AR determination |
| 11 | Incr | Incr | N/C | No | BP & StO2 changes not likely related or relationship cannot be determined, StO2 possibly affected by other physiological process (hyperoxia) | Physiology filter: hyperoxia event confounder to AR determination |

*FIG. 29*

EVENT

baseline

ΔHHb

ΔrTHb

ΔO2Hb

(ΔO2Hb-ΔHHb)
StO$_2$ %

INDICATIONS:

Respiratory Distress:
- Lung Failure;
- Irregular Breathing Pattern;
- Low Oxygen Environment;

Cardiovascular Deficiency:
- Moderate ↓ HR, P$_{art}$; ("P$_{art}$" = Arterial pressure)

*FIG. 30*

(ΔO2Hb-ΔHHb)
StO$_2$ %

EVENT

baseline

ΔO2Hb

ΔrTHb

ΔHHb

INDICATIONS:

Respiratory Recovery:
- Improved Lung Function;
- Resume Normal Breathing Patterns;
- Increased Oxygen Environment;

Cardiovascular Recovery:
- Moderate ↑ HR, P$_{art}$; ("P$_{art}$" = Arterial pressure)

*FIG. 31*

EVENT

baseline

ΔHHb

ΔrTHb

ΔO2Hb

(ΔO2Hb-ΔHHb)
StO₂ %

INDICATIONS:

Cardiovascular Deficiency:
- Severe ↓ HR, Part, Stroke volume;
- ↓ Cerebral blood flow, Perfusion pressure;
- Arrhythmia;
- Vasoconstriction, Hypocapnia;
- Shock;
- Hemodilution (bypass)

*FIG. 32*

(ΔO2Hb-ΔHHb)
StO₂ %
ΔO2Hb
ΔrTHb

EVENT

baseline

ΔHHb

INDICATIONS:

Cardiovascular Recovery
- Improved Heart Function;
- Hyperemic Response After Ischemia;
- ↑ Cerebral Blood Flow;

Other:
- Brain Center Activation (small change)
- Vasodilation, Hypercapnia

*FIG. 33*

EVENT

baseline

ΔrTHb

ΔHHb

ΔO2Hb

(ΔO2Hb-ΔHHb)
StO₂ %

**Pattern E:**

INDICATIONS:

Venous Congestion / Pooling / Impaired Venous Return:
- StO2 and THb trend in opposite direction;

*FIG. 34*

Sample data window
20 sec to 5 minutes

NIRS StO2
(O2Hb - HHb)
rTotalHb

Trending filter:
slope (1) x
slope (2) =
positive value
(= True)

Standard
deviation of
parameters >
threshold
(= True)

AND Gate
Function

TRUE:
Data valid for
Autoregulation
Determination
(data binned)

*FIG. 35*

BP slope >0 (=True) ⟹

HR slope >0 (=True) ⟹

AND Gate Function ⟹

TRUE:
Data NOT valid for
Autoregulation
Determination
(data NOT binned)

*FIG. 36*

Heart Rate ⟹
BP systolic ⟹
Multi-Band COHZ ⟹
Polarity Filter &
Threshold
Change
Qualifier ⟹

COHZ (C1) HR:BP

$f(C1,C2)$ =
"AND"
C1 + C2,
etc.
Qualify pain
events ⟹

Pain /
Nociception
Event
(+intensity)

StO2 or ΔHbD ⟹
Heart Rate
(EEG)? ⟹
Multi-Band COHZ ⟹
Polarity Filter &
Threshold
Change
Qualifier ⟹

COHZ (C2) StO2:HR

*FIG. 37*

Heart Rate ⟹ | Multi-Band COHZ | ⟹ | Polarity Filter & Threshold Change Qualifier | ⟹ COHZ (C1) HR:BP
BP systolic ⟹

$f$(C1,C2) = "AND" C1 + C2 + C3, etc. Qualify pain events ⟹ Pain / Nociception Event (+intensity)

StO2 or ΔHbD ⟹ | Multi-Band COHZ | ⟹ | Polarity Filter & Threshold Change Qualifier | ⟹
Heart Rate ⟹

rTHb ⟹ | Multi-Band COHZ | ⟹ | Polarity Filter & Threshold Change Qualifier | ⟹
Heart Rate ⟹

COHZ (C2) StO2:HR

COHZ (C3) rTHb:HR

*FIG. 38*

*FIG. 39A*

*FIG. 39B*

*FIG. 40*

*FIG. 41*

FIG. 42

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63189813
- US 63231463
- US 6456862 B
- US 7072701 B
- US 8078250 B
- US 8396526 B
- US 8965472 B

- US 10117610 B
- US 9913601 B
- US 9848808 B
- US 9456773 B
- US 9364175 B
- US 9923943 B
- US 8788004 B

**Non-patent literature cited in the description**

- Finding a needle in a haystack: Detecting knee points in system behavior. **SATOPAA et al.** 2011 31st International Conference on Distributed Computing Systems Workshops. IEEE, 2011